(19) **Europäisches Patentamt / European Patent Office / Office européen des brevets**

(11) **EP 3 084 427 B1**

(12) **EUROPEAN PATENT SPECIFICATION**

(45) Date of publication and mention of the grant of the patent:
**12.09.2018 Bulletin 2018/37**

(21) Application number: **14815828.0**

(22) Date of filing: **18.12.2014**

(51) Int Cl.:
**G01N 33/487** (2006.01)

(86) International application number:
**PCT/GB2014/053754**

(87) International publication number:
**WO 2015/092411 (25.06.2015 Gazette 2015/25)**

(54) **NANOPORE ARRANGEMENT FOR DNA SEQUENCING**

NANOPORENANORDNUNG ZUR DNA-SEQUENZIERUNG

ORGANISATION DE NANOPORES DE SÉQUENÇAGE D'ADN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2013 GB 201322399**

(43) Date of publication of application:
**26.10.2016 Bulletin 2016/43**

(73) Proprietor: **Lancaster University Business Enterprises Limited**
**Lancaster LA1 4YW (GB)**

(72) Inventors:
- **SADEGHI, Hatef**
  **Lancaster LA1 4YB (GB)**
- **LAMBERT, Colin John**
  **Lancaster LA1 4YB (GB)**
- **ALGHARAGHOLY, Laith A. A.**
  **Lancaster LA1 4YB (GB)**
- **BAILEY, Steven William Dennis**
  **Lancaster LA1 4YB (GB)**
- **RODRÍGUEZ, Jaime Ferrer**
  **E-33007 Oviedo (ES)**
- **SUAREZ, Victor Manuel Garcia**
  **E-33007 Oviedo (ES)**

(74) Representative: **Stevenson-Hill, Jack Patrick**
**Marks & Clerk LLP**
**1 New York Street**
**Manchester M1 4HD (GB)**

(56) References cited:
**US-A1- 2010 327 847**

- LIANG QI ET AL: "Slip corona surrounding bilayer graphene nanopore", NANOSCALE, vol. 4, no. 19, 17 July 2012 (2012-07-17), page 5989, XP055172465, ISSN: 2040-3364, DOI: 10.1039/c2nr31405c
- Y. HE ET AL: "DNA Sequencing with nanopore-embedded bilayer-graphene nanoelectrodes", 2010 10TH IEEE INTERNATIONAL CONFERENCE ON SOLID-STATE AND INTEGRATED CIRCUIT TECHNOLOGY, 1 November 2010 (2010-11-01), - 4 November 2010 (2010-11-04), pages 1483-1485, XP055172466, DOI: 10.1109/ICSICT.2010.5667534 ISBN: 978-1-42-445797-7
- LAITH ALGHARAGHOLY ET AL: "Sculpting molecular structures from bilayer graphene and other materials", PHYSICAL REVIEW B, vol. 86, no. 7, 13 August 2012 (2012-08-13), XP055172445, ISSN: 1098-0121, DOI: 10.1103/PhysRevB.86.075427
- HATEF SADEGHI ET AL: "Graphene Sculpturene Nanopores for DNA Nucleobase Sensing", THE JOURNAL OF PHYSICAL CHEMISTRY B, vol. 118, no. 24, 21 May 2014 (2014-05-21), pages 6908-6914, XP055172435, ISSN: 1520-6106, DOI: 10.1021/jp5034917

**Description**

This invention relates to an electrical sensor assembly, a sensor arrangement including an electrical sensor assembly, and a method of producing an electrical sensor assembly from a multilayer structure.

Deoxyribonucleic Acid (DNA) is a well-known molecule that encodes genetic information in living organisms.

Most DNA molecules are double stranded helical structures, each strand comprising a long biopolymer. The two biopolymer strands of DNA are bound non-covalently together. The double stranded DNA structure can be separated into two single stranded DNA molecules by various methods including mechanically, by the use of high temperature, by the use of a low salt environment, and by the use of a high pH environment.

Each biopolymer is made up of a large number of units, referred to as nucleotides, which are joined to one another in end-to-end fashion. Each nucleotide comprises a nucleobase and a backbone. It is the backbone of each nucleotide which is joined to the backbone of adjacent nucleotides in an end-to-end fashion. The backbone is made of alternating sugars and phosphate groups.

The nucleobase of each nucleotide is attached to a sugar of the backbone. There are four different types of nucleobase - guanine, adenine, thymine, and cytosine (commonly referred to as G, A, T, C respectively). It is the sequence of nucleobases of each nucleotide along the strands of the DNA molecule that encodes genetic information. A DNA molecule may be a very large molecule containing many millions of nucleotides.

Advances in medical genetics have provided a more detailed understanding of the effect of genetics in disease. That is to say, there is a greater understanding that the genetic information encoded in DNA may affect disease which is experienced by an organism possessing the DNA. In order to categorise the genetic information encoded within a DNA molecule, and hence study its possible effect on disease, is important to determine the type and order of nucleotides within a DNA molecule. Such a determination of the order of nucleotides within DNA is referred to as DNA sequencing.

There are several known methods for sequencing DNA.

Known methods of DNA sequencing include Maxam-Gilbert sequencing and Sanger sequencing. Maxam-Gilbert and Sanger sequencing involve different chemical and radioactive labelling processes to arrive at a gel which can be electrophoresed and then exposed to an X-ray film to produce a contrast pattern which is representative of the structure of the DNA (and hence the sequence of the nucleotides within the DNA).

These methods of DNA sequencing have been used for over 30 years. Despite the fact that these processes have now been automated, they are still relatively slow and expensive.

Recently, various other methods have been developed in order to attempt to achieve DNA sequencing methods which are as accurate as those used in the past, but which are faster and less expensive. Examples of such methods include single-molecule real-time sequencing, ion semiconductor sequencing, 454 pyrosequencing, sequencing by synthesis, and sequencing by ligation. Other methods of DNA sequencing are also being developed. These include sequencing by hybridisation, sequencing using mass spectrometry, micro-fluidic Sanger sequencing, microscopy-based sequencing, and nanopore sequencing.

Nanopore sequencing is a method of DNA sequencing whereby a strand of DNA is passed through a small hole (a nanopore) and a change in a particular electrical signal is measured whilst the DNA strand passes through the nanopore. This is discussed in more detail below. In all cases to date, the solid material containing the nanopore is electrically insulating.

As previously discussed, a nanopore is simply a very small hole. The nanopore may have an internal diameter of the order of one nanometre. The internal diameter of the nanopore is, of course, large enough such that the species to be analysed by the nanopore can pass through the nanopore.

Various ways of creating a nanopore are known. Nanopores can be formed in synthetic materials such as silicon nitride or graphene. The forming of nanopores in such materials may be achieved, for example, by drilling through the synthetic material using transmission electron microscopy (TEM). Nanopores formed in appropriate synthetic materials may be referred to as solid-state nanopores. Another known way of creating a nanopore is the formation of a nanopore using a pore-forming protein in a membrane such as a lipid bi-layer. An example of a known pore-forming protein is $\alpha$-haemolysin. Furthermore, there are also hybrid nanopores which are formed by locating a pore-forming protein within a suitable aperture in synthetic material.

As previously discussed, nanopores can be formed out of graphene. Graphene is an allotrope of carbon. Its structure is that of single atom thickness planar sheets of $sp^2$-bonded carbon atoms packed in a two-dimensional honeycomb crystal lattice. Each carbon atom within the lattice is found at the intersection of three adjacent six-membered rings.

Once the nanopore has been fabricated, in order to carry out nanopore sequencing the nanopore is immersed in a conducting fluid (for example an ionic solution). Electrodes in the conducting fluid are used to establish a potential difference (voltage) between the conducting fluid on one side of the nanopore and the conducting fluid on the other side of the nanopore. In order to sequence a DNA molecule the electric current due to conduction of ions passing through the nanopore from the conducting fluid on one side of the nanopore to the conducting fluid on the other side of the nanopore is measured. As a result of each of the nucleotides of the DNA strand having a different configuration (due to the different configurations of each of the nucleobases) each of the different types of nucleotide of the DNA strand modifies the flow rate of the conduction ions through the nanopore in a different way. For example, each of the different types of nucleotide may block the flow of conduction ions through the nanopore to a different extent. The difference in modification of the flow rate of conduction ions through the nanopore for different types of nucleotide results in a different, characteristic, electric current flowing between the electrodes as each type of nucleotide passes through the nanopore. To optimise the signal, the material containing the nanopore is chosen to be electrically insulating, so that the electrical current passes primarily through the nanopore.

Consequently, nanopore sequencing works by passing a DNA strand through the nanopore in a particular direction and measuring the change in current flow in between the electrodes either side of the nanopore in order to determine the order of nucleotides which pass through the nanopore, and hence the order of nucleotides within the DNA strand.

US2010/327847 relates to a solid state molecular sensor which has an aperture extending through a thickness of a sensing region. The sensing region can be a region of graphene made of one or more layers of graphene and including an aperture extending through a thickness of the graphene.

Currently known nanopore sequencing techniques suffer from several problems.

First, in some cases the current flowing through the nanopore when each of the types of nucleotide passes through the nanopore is fairly similar to the extent that certain systems have not had the sensitivity required to distinguish between the different types of nucleotide.

Secondly, the requirement of a conducting fluid with electrodes located within the conducting fluid either side of the nanopore means that the apparatus required for nanopore sequencing may be relatively complex, bulky, and expensive.

Thirdly, measurement of ionic current flowing through the nanopore leads to an inherently slow measurement process, because measurement is limited by the transport speed of the charge carriers (e.g. ions within an ionic solution).

If a biological nanopore is used, then such a nanopore may be easily denatured in certain environments (for example extremes of temperature or pH). If the nanopore is denatured, then the flow characteristics of the conducting fluid through the nanopore change, which may result in erroneous measurements. Furthermore, the nanopore may denature to an extent that the DNA can no longer pass through it.

Finally, in the case of solid-state nanopores, if the nanopore is formed in a thin (e.g. single layer) solid-state material, then the edges of the solid state material which define the nanopore may be unstable. For example, if a hole is cut in single-layer graphene, then the carbon atoms at the edge of the nanopore may include unstable "dangling bonds". In this case, edge atoms which define the nanopore may be lost over time or when certain materials pass through the nanopore. Equally, in such situations, the edge atoms defining the nanopore may reconfigure their bonding. In such examples, the geometry of the nanopore becomes altered, which, again, may result in a change in flow of the conducting liquid through the nanopore thereby affecting the measured current through the nanopore and potentially resulting in erroneous readings.

It is an object to the present invention to provide an electrical sensor assembly suitable for measuring a property of a chemical species, for example identifying nucleotides within a DNA strand, which obviates or mitigates problems within the prior art, whether discussed above or otherwise. It is a further object of the present invention to provide an alternative electrical sensor assembly. It is a still further object of the present invention to provide a sensor arrangement including an electrical sensor assembly as previously discussed. Finally, it is an objective to the present invention to provide a method of manufacturing an electrical sensor assembly as previously discussed.

According to a first aspect of the present invention there is provided a sensor arrangement comprising an electrical sensor assembly suitable for measuring a property of a chemical species, the electrical sensor assembly comprising first and second electrodes comprising first and second generally planar molecular layers, the first and second generally planar molecular layers each consisting of an array of covalently bonded atoms; and wherein the first molecular layer is covalently bonded to the second molecular layer to define an aperture through both the first and second molecular layers; and wherein the aperture is configured to enable the chemical species to pass through the aperture; and the sensor arrangement further comprising an electrical power supply connected to the first electrode and the second electrode, wherein the power supply is configured to apply a voltage across the first electrode and second electrode.

The electrical sensor assembly comprises first and second electrodes comprising first and second generally planar molecular layers. In some embodiments the first and second electrodes may each comprise first and second generally planar molecular layers. In other embodiments the first electrode may comprise the first generally planar molecular layer and not the second generally planar molecular layer, and the second electrode may comprise the second generally planar molecular layer and not the first generally planar molecular layer. In further other embodiments, the first electrode may comprise both the first and second generally planar molecular layers, and the second electrode may comprise the first generally planar molecular layer and not the second generally planar molecular layer.

The sensor arrangement further comprises a current measuring device configured to measure current flow between the first and second electrodes.

The first electrode may comprise the first molecular layer and the second electrode may comprise the second molecular layer, wherein the first electrode generally extends in a first direction away from the aperture and away from the second electrode, and wherein the current flow between the first and second electrodes flows at least partially via the covalent bonding between the first and second molecular layers.

According to a second aspect of the present invention there is provided a method of measuring a property of a chemical species using an electrical sensor assembly, the electrical sensor assembly comprising first and second electrodes comprising first and second generally planar molecular layers, the first and second generally planar molecular layers each consisting of an array of covalently bonded atoms; and wherein the first molecular layer is covalently bonded to the second molecular layer to define an aperture through both the first and second molecular layers; and; the method comprising passing the chemical species through the aperture; applying a voltage across the first and second electrodes; and measuring current flow between the first and second electrodes.

The electrical sensor assembly comprises first and second electrodes comprising first and second generally planar molecular layers. In some embodiments the first and second electrodes may each comprise first and second generally planar molecular layers. In other embodiments the first electrode may comprise the first generally planar molecular layer and not the second generally planar molecular layer, and the second electrode may comprise the second generally planar molecular layer and not the first generally planar molecular layer. In further other embodiments, the first electrode may comprise both the first and second generally planar molecular layers, and the second electrode may comprise the first generally planar molecular layer and not the second generally planar molecular layer.

The first electrode may comprise the first molecular layer and the second electrode may comprise the second molecular layer, wherein the first electrode generally extends in a first direction away from the aperture and away from the second electrode, and wherein the current flow between the first and second electrodes flows at least partially via the covalent bonding between the first and second molecular layers.

The first electrode may comprise the first generally planar molecular layer and not the second generally planar molecular layer, and the second electrode may comprise the second generally planar molecular layer and not the first generally planar molecular layer.

The following statements apply to any of the aspects of the present invention.

The array of covalently bonded atoms of one or both of the first and second generally planar molecular layers may be a repeating structure, the repeating structure repeating in two substantially perpendicular directions.

The first and/or second generally planar molecular layers may be one atom thick.

At least one of the first and second generally planar molecular layers may be a graphene layer.

At least one of the first and second generally planar molecular layers may be a silicene layer.

The first and second generally planar molecular layers may have substantially the same composition and/or structure. The first and second generally planar molecular layers may be graphene layers. The first and second generally planar molecular layers may be silicene layers.

The first and second generally planar molecular layers may have different compositions and/or structures.

The chemical species may be a polymer with inhomogeneous charge distribution along its length.

The chemical species may be DNA.

The first electrode may be covalently bonded to the second electrode by $sp^2$ covalent bonding. In some embodiments the first electrode may be covalently bonded to the second electrode In the vicinity of the aperture by $sp^2$ covalent bonding.

According to a third aspect of the present invention there is provided a DNA sequencing device comprising a sensor arrangement according to the first aspect of the invention.

According to a fourth aspect of the present invention there is provided a method of DNA sequencing comprising a method according to the second aspect of the invention.

Within all the aspects of the invention discussed above, where reference is made to the measurement of current flow, for example, between the first and second electrodes, it will be appreciated that such current flow is may an electron current flow (in other words, current due to the flow of charge carriers in the form of electrons is measured). For example, in the case where current flow is measured between the first and second electrodes, the current flow of electrons between the first and second electrodes may be measured. This differs from known methods and apparatus for DNA sequencing, in which measured current flow is generally the current flow of ions through an aperture (or pore) of an electrical sensor assembly (in other words, current due to the flow of charge carriers in the form of ions through the aperture is measured). The current flow of ions in known methods and apparatus for DNA sequencing may include the flow of ions exterior to the device. In the present invention an electron current within the sensor device itself may be measured.

Specific embodiments of the present invention will now be described, by way of example only, with reference to the accompanying drawings in which:

Figure 1 shows a schematic side view of a portion of a known DNA sequencing device;

Figure 2 shows schematic views of the structure of the four nucleobases of DNA;

Figure 3 shows a schematic isometric view of a portion of a single layer of graphene;

Figure 4 shows a schematic plan view of a portion of a single layer of graphene;

Figure 5 shows schematic plan, side and front views of the structure of an electrical sensor assembly and sensor arrangement in accordance with embodiments of the present invention;

Figure 6 shows schematic plan, side and front views of the structure of an electrical sensor assembly and sensor arrangement in accordance with further embodiments of the present invention;

Figure 7 shows a schematic view of the structure of a portion of bilayer graphene; 67451941-1-JSHILL

Figure 8 shows a schematic side view of a cutter being used to create a first edge in a first molecular layer and a second edge in a second molecular layer, the first and second edges being able to covalently bond to one another to define part of an aperture of an electrical sensor assembly in accordance with an embodiment of the present invention;

Figures 8a and 8b show a plan view and a perspective view respectively of a portion of AB-stacked bilayer graphene which has been cut by a cutter in order to produce an aperture as shown in Figures 5 and 6;

Figure 9A shows a plot of transmission coefficient against charge carrier energy for the electrical sensor assembly shown in Figure 5, with no chemical species received by the aperture of the electrical sensor assembly;

Figure 9B shows a plot of the differential transmission coefficient against charge carrier energy for each of the four nucleobases of DNA when they are each received by the aperture of the electrical sensor assembly shown in Figure 5;

Figure 10 shows a plot of transmission coefficient against charge carrier energy for the electrical sensor assembly shown in Figure 6, with no chemical species received by the aperture of the electrical sensor assembly;

Figure 11 shows schematic plan, side and front views to the electrical sensor assembly shown in Figure 6 whilst it receives an Adenine nucleobase, and a plot of the differential transmission coefficient against charge carrier energy in the case where Adenine is received by the aperture of the electrical sensor assembly shown in Figure 6;

Figure 12 shows schematic plan, side and front views to the electrical sensor assembly shown in Figure 6 whilst it receives a Cytosine nucleobase, and a plot of the differential transmission coefficient against charge carrier energy in the case where Cytosine is received by the aperture of the electrical sensor assembly shown in Figure 6;

Figure 13 shows schematic plan, side and front views to the electrical sensor assembly shown in Figure 6 whilst it receives a Guanine nucleobase, and a plot of the differential transmission coefficient against charge carrier energy in the case where Guanine is received by the aperture of the electrical sensor assembly shown in Figure 6;

Figure 14 shows schematic plan, side and front views to the electrical sensor assembly shown in Figure 6 whilst it receives a Thymine nucleobase, and a plot of the differential transmission coefficient against charge carrier energy in the case where Thymine is received by the aperture of the electrical sensor assembly shown in Figure 6;

Figure 15 shows the probability distribution $P_x$ of the set $\{\alpha_x(E)\}$ defined in equation 10 for each of four nucleobases when they are received by the electrical sensor assembly shown in Figure 6, the distribution being taken over four different relative orientations between the relevant nucleobase and the electrical sensor assembly; and

Figure 16 shows the probability distribution $P_X(\beta)$ of the set $\{\beta_{X,m}\}$ defined in equation 15 for each of four nucleobases.

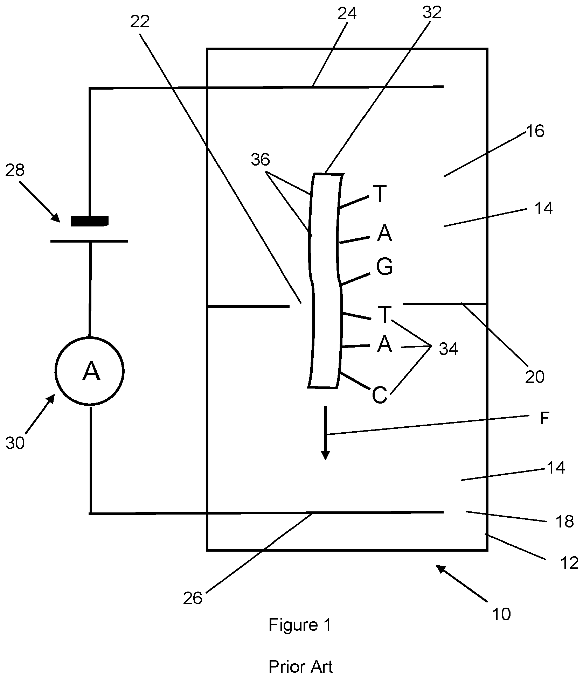

Figure 1 shows a schematic view of a known DNA sequencing apparatus. The apparatus 10 includes a vessel 12 filled with an ionic (and hence electrically conductive) liquid 14. The vessel 12 is split into a first section 16 and a second section 18 by a dividing wall 20. The dividing wall includes an aperture 22 which enables the fluid 14 to flow between the first and second sections 16, 18 of the vessel 12.

The aperture 22 in the dividing wall 20 has a diameter in the nanometre range. Consequently, the aperture 22 may be said to be a nanopore. The diameter of the nanopore 22 may be any appropriate diameter, for example, but not limited to, about 0.1 nm to about 3 nm.

A first electrode 24 is located within the liquid 14 in the first section 16 of the vessel 12. A second electrode 26 is located in the liquid 14 within the second section 18 of the vessel 12. It may be said that the first electrode 24 is on a first side of the nanopore 22, whereas the second electrode 26 is on a second side of the nanopore 22. Put another way, the first electrode 24 is one side of the nanopore 22 and the second electrode 26 is on the other side of the nanopore 22.

The electrodes 24, 26 are connected to a power supply 28. The power supply 28 is configured to generate a voltage, i.e. generate a potential difference between the first electrode 24 and the second electrode 26.

A current measuring device 30 is also connected between the first and second electrodes 24, 26. The current measuring device 30 is configured to measure the flow of charge between the first and second electrodes 24, 26.

As previously discussed, the liquid 14 is an ionic liquid; that is to say, it contains ions. Because the ions within the liquid are free to move within the liquid, the application of a voltage between electrodes 24 and 26 causes the ions within the liquid 14 to flow between the first electrode 24 and the second electrode 26. The direction of the flow of ions (and hence the direction of the current flowing between the electrodes 24 and 26) depends on the relative polarity of the electrodes 24 and 26 as is well understood by those skilled in the art. In the embodiment shown in Figure 1, the power supply 28 generates a voltage at the electrodes 24 and 26 which causes ions within the liquid 14 to flow in the direction of the arrow F.

Within the embodiment shown in Figure 1 it can be seen that a DNA strand 32 is located within the nanopore 22. The DNA strand, as previously discussed, is made up of several nucleotides. Each nucleotide includes a nucleobase 34 and a backbone portion 36. Each nucleotide within the DNA strand is joined to at least one (and usually two) adjacent nucleotides by the backbone portion 36 of the nucleotide being joined to the backbone portion of at least one (and usually two) backbone portions of adjacent nucleotides.

The flow F of ions within the liquid 14 causes ions within the liquid to flow from the first section 16 of the vessel 12 to the second section 18 of the vessel 12 via the nanopore 22. An electric field within the liquid 14 causes the DNA strand 32 to be moved through the nanopore 22 by the interaction of the DNA strand 32 with the moving ions.

At any given time whilst the DNA strand 32 is received by the nanopore 22, a portion of the DNA strand 32 and ions within the liquid 14 are simultaneously moving through the aperture 22. The flow rate at any given time of ions between the electrodes 24 and 26, and hence through the aperture 22, determines the current flowing from the electrode 24 to the electrode 26 and hence the current measured by the current measuring device 30.

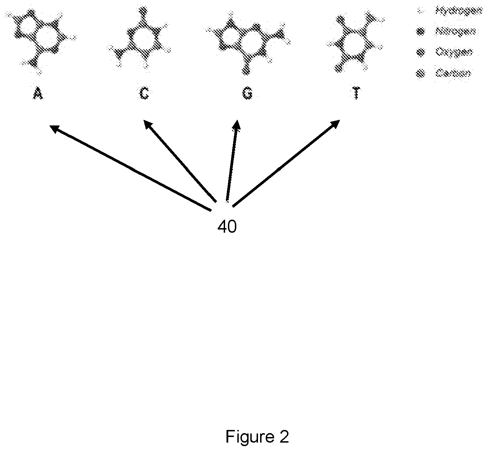

As shown in Figure 2, the nucleobases 40 of each type of nucleotide of DNA each have a different structure and therefore shape. As each of the nucleobases of a DNA strand passes through the nanopore 22 (each nucleobase being attached to a backbone as previously discussed), it modifies the flow of ions through the nanopore 22 at that time. A consequence of the different shapes of the four types of nucleobase is that each type of nucleobase modifies the flow of ions through the nanopore 22 to a different extent. Consequently, the current flowing between the electrodes 24 and 26 (and hence the current measured by the current measuring device 30) is different for each type of nucleotide passing through the nanopore 22. As a result, by measuring the current between the electrodes 24 and 26 using the current measuring device 30 as a strand of DNA 32 passes through the nanopore 22, it is possible to identify the type and order of nucleotides passing through the nanopore 22, and thereby sequence the DNA strand 32.

There are various different known ways of fabricating a nanopore. Some nanopores may be formed using a pore-forming protein in a membrane. Another way of forming a nanopore is to fabricate a solid-state nanopore.

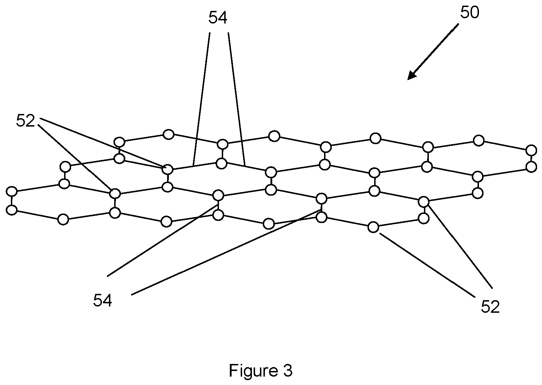

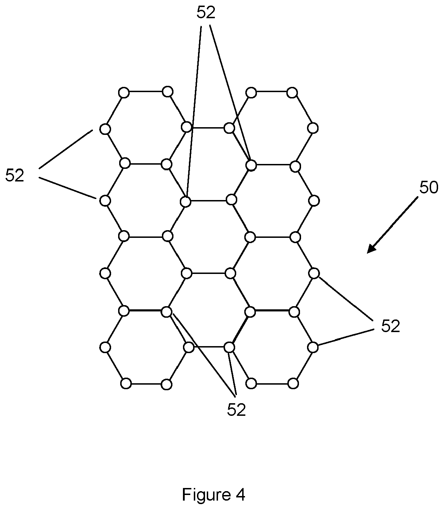

Figures 3 and 4 show schematic perspective and plan views respectively of the structure of a portion of a single layer of graphene. A single layer of graphene may also be referred to as a graphene monolayer or a monolayer graphene sheet. The monolayer graphene sheet 50 is a planar molecular layer consisting of an array of covalently bonded carbon atoms 52. Each carbon atom 52 (other than those at the edges of the sheet) is connected by three respective covalent bonds 54 to three adjacent carbon atoms 52. The covalent bonds 54 are $sp^2$ covalent bonds. The structure of the sheet 50 is such that the carbon atoms 52 are packed in a two-dimensional honeycomb crystal lattice (i.e., consisting of tessellated hexagons or hexagonal rings).

In order to create the nanopore, the nanopore may be cut into a graphene sheet using any appropriate method, for example using Transmission Electron Microscopy (TEM) (discussed in, for example, S. Liu, Q. Zhao, J. Xu, K. Yan, H. Peng, F. Yang, et al., "Fast and controllable fabrication of suspended graphene nanopore devices," Nanotechnology, vol. 23, pp. 085301-085301, March 2 2012) or cold ion beam sculpting (discussed in, for example, A. T. Kuan and J. A. Golovchenko, "Nanometer-thin solid-state nanopores by cold ion beam sculpting," Applied Physics Letters, vol. 100, May 21 2012) techniques.

Nanopore DNA sequencing devices such as that described above, which include a nanopore fabricated from a single graphene sheet, have several disadvantages. First, the speed at which such a device can sequence DNA is inherently limited by the fact that it is dependent upon the transport speed of the charge carriers (in this case ions within the liquid). Secondly, cutting an aperture (i.e. the nanopore) into a graphene sheet can leave various 'dangling' bonds of the carbon atoms which define the edge of the nanopore. These dangling bonds may lead to instability in the bonding of carbon atoms at the edge of the nanopore relative to the rest of the graphene sheet. The instability in bonding of the carbon atoms at the edge of the nanopore may lead to carbon atoms at the edge of the nanopore breaking free from the rest of the graphene sheet. In this case, the dimensions of the nanopore change (i.e. the nanopore becomes bigger). Changing the size of the nanopore of course affects the rate at which ions within the liquid can pass through the nanopore (i.e. the rate increases). Consequently, a change in the size of the nanopore results in a change in the current measured by the current measuring device and may hence lead to an incorrect determination as to which nucleotide is passing through the nanopore at any given time. Thirdly, the requirement of ionic liquid and electrodes in order to carry out the sequencing leads to the relatively high complexity and relatively high cost of the sequencing device.

The current invention provides a novel and inventive sensor arrangement which may be used for DNA sequencing.

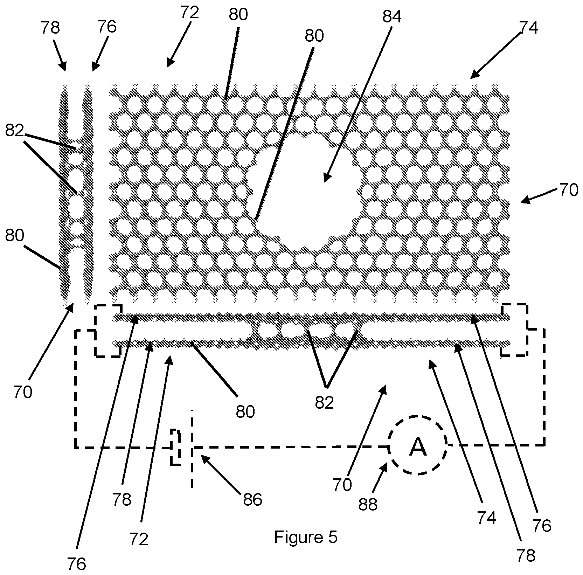

Figure 5 shows a schematic plan, side and front views of a sensor arrangement in accordance with an embodiment of the present invention which is suitable for measuring a property of a chemical species (for example identifying a sequence of nucleotides within a strand of DNA). The electrical sensor assembly 70 comprises first and second electrodes 72, 74 comprising first and second generally planar molecular layers 76, 78. The first and second generally planar molecular layers 76, 78 each consist of an array of covalently bonded atoms (some of which are indicated with the reference numeral 80 within the Figure). The first molecular layer is covalently bonded (see for example bonds indicated within the Figure by reference numeral 82) to the second molecular layer 78 to define an aperture 84 through both the first and second molecular layers 76, 78. The aperture is configured to enable the chemical species of which the sensor assembly is measuring a property to pass through the aperture 84. For example, the aperture 84 may have a size (e.g. diameter) and/or shape such that the chemical species may pass through it.

The sensor arrangement further comprises an electrical power supply 86 (indicated in dashed lines within Figure 5). The electrical power supply 86 that is connected to the first electrode 72 and the second electrode 74 and is configured to apply a voltage across the first electrode 72 and second electrode 74. It will be appreciated that any appropriate electrical power supply may be used in order to apply a voltage across the first and second electrodes 72, 74. Many suitable electrical power supplies are known in the art and, as such, further discussion of this portion of the sensor arrangement is omitted. The sensor arrangement further comprises a current measuring device 88 configured to measure current flow between the first and second electrodes. Again, it will be appreciated that any appropriate current measuring device may be used in order to measure the current flow between the first and second electrodes 72, 74. Many suitable current measuring devices are known in the art and, as such, again, further discussion of this portion of the sensor arrangement is omitted.

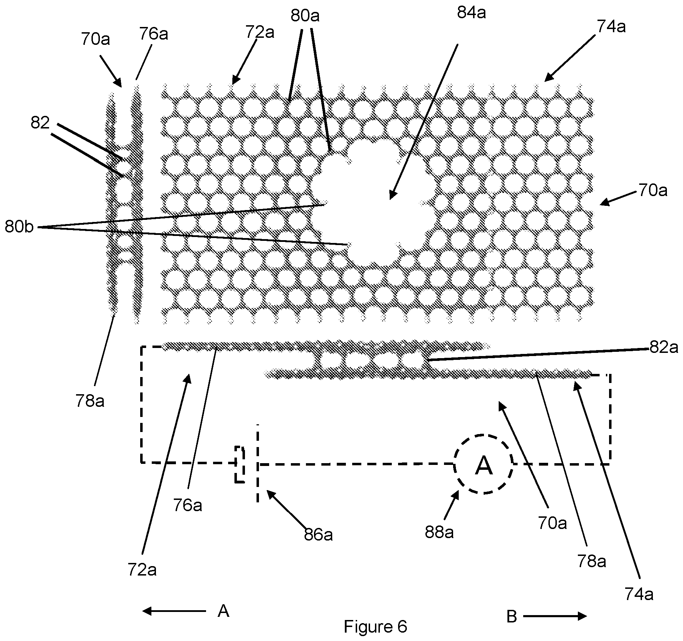

Figure 6 shows a schematic plan, side and front views of a sensor arrangement in accordance with an embodiment of the present invention. The electrical sensor assembly shown in Figure 6 is similar to that shown in Figure 5 and is also suitable for measuring a property of a chemical species. The electrical sensor assembly 70a has first and second electrodes 72a, 74a which comprise respective first and second generally planar molecular layers 76a, 78a. That is to say, the first electrode 72a comprises the first molecular layer 76a, and the second electrode 74a comprises the second molecular layer 78a. As previously discussed, the first and second generally planar molecular layers 76a, 78a each consist of an array of covalently bonded atoms 80a.

The first molecular layer 76a of the first electrode 72a is covalently bonded to the second molecular layer 78a of the second electrode 74a to define an aperture 84a through both the first and second molecular layers 76a, 78a. Again, the aperture 84a is configured to enable the chemical species, the property of which may be measured by the electrical sensor assembly, to pass through the aperture 84a. For example, the aperture 84a may have a size (e.g. diameter) and/or shape such that the chemical species may pass through it.

The first electrode 72a generally extends in a first direction A away from the aperture 84a and away from the second electrode 74a. Likewise, in this embodiment, the second electrode 74a generally extends in a second direction B away from the aperture 84 and away from the first electrode 72a. In this embodiment the first direction A is opposite to the second direction B.

It will be appreciated that, in other embodiments, the configuration of the first and second electrodes 72a, 74a may be different. For example, the length of the electrodes (i.e. the distance between the aperture and the end of the electrode furthest away from the aperture) may be any appropriate length. For example, the lengths of the first and second electrodes may be different. Furthermore, although it is preferred that the first and second electrodes extend in directions which are opposite to one another (i.e. such that the first and second electrodes 72a, 74a are diametrically opposed from one another about the aperture), in other embodiments this need not be the case. For example, in the embodiment shown, the angle subtended between direction A and direction B in a plane which is substantially parallel to the planes of the molecular layers is approximately 180°. In other embodiments the angle between the first and second directions may be any appropriate angle.

The electrical sensor assembly 70a shown in Figure 6 forms part of the sensor arrangement. The sensor arrangement (shown in dotted lines in Figure 6) includes an electrical power supply 86a connected to the first electrode 72a and second electrode 74a. The power supply is configured to apply a voltage across the first electrode 72a and second electrode 74a. The sensor arrangement also includes a current measuring device 88a which is configured to measure current flow between the first and second electrode 72a, 74a via the covalent bonding 82a between the first and second electrodes 72a, 74a. Unlike in the embodiment shown in Figure 5, in the embodiment shown in Figure 6 the first electrode is only joined to the second electrode by covalent bonding between the first molecular layer (of the first electrode) and the second molecular layer (of the second electrode). As such, current flowing between the first and second electrode 72a, 74a only flows via the covalent bonding 82a between the first and second electrodes 72a, 74a that define the aperture 84a. Again, it will be appreciated that any appropriate electrical power supply and/or current measuring device may be used.

Because the first electrode is only joined to the second electrode by covalent bonding between the first molecular layer (of the first electrode) and the second molecular layer (of the second electrode), when a voltage is applied across the first and second electrodes, current can only flow between the first and second electrodes via the covalent bonding which defines the aperture. Consequently, the charge carriers which carry the current between the first and second electrodes are more likely to interact with a chemical species (or part thereof) which is received by the aperture. Consequently, this embodiment of the invention is more sensitive to a chemical species (or part thereof) received by the aperture than, say, the embodiment shown in Figure 5. Consequently, the embodiment in Figure 6 may be better suited to measuring a property of a chemical species received by the aperture, for example, identifying nucleotides within a strand of DNA.

The embodiment of electrical sensor assembly shown in Figure 6 also differs from that shown in Figure 5 in that some of the atoms which define the edge of the aperture also include a covalent bond to a hydrogen atom (e.g. atoms indicated by 80b). This hydrogen could be bonded to the edge of the aperture by heating the pore in an atmosphere of hydrogen. The incorporation of hydrogen may also increase the stability of the pore.

It will be appreciated that electrical senor assemblies according to embodiments of the invention may or may not include hydrogen bonded at the edge of the aperture. For example one embodiment of the invention may be the same as that shown in Figure 5 with hydrogen bonded, and another embodiment of the invention may be the same as that shown in Figure 6, but without hydrogen bonded.

Within each of the electrical sensor assemblies and sensor arrangements shown in Figures 5 and 6, the first and second generally planar molecular layers are graphene layers. The first and second adjacent generally planar molecular layers are AA-stacked. Furthermore, each of the first and second generally planar molecular layers is one atom thick. Finally, in the vicinity of the pore, the first electrode is covalently bonded to the second electrode by $sp^2$ covalent bonding.

In other embodiments of the invention, this need not be the case. For example, the array of covalently bonded atoms in one or both of the first and second generally planar molecular layers may be any repeating structure, the repeating structure repeating in two substantially perpendicular directions. Furthermore, any first and second generally planar molecular layer which is one atom thick may be used. It will be appreciated that in other embodiments, any appropriate thickness of generally planar molecular layer may be used.

Although both the first and second molecular layers within the described embodiments are graphene layers, in other embodiments the generally planar molecular layers may be formed from any appropriate material. For example, one of the molecular layers may be a graphene layer and the other molecular layer may be formed of a different material. Alternatively, in some embodiments, neither of the first and second molecular layers need be a graphene layer. Other examples of suitable materials include conducting and semi-conducting materials such as Silicene, Silicane, Germanene, Germanane, Molybdenum disulfide, Gallium selenide, Bismuth telluride, and single layer crystals including formed from appropriate combinations of group III and IV atoms. It will be appreciated that the first and second molecular layers may have substantially the same composition and/or structure in some embodiments, and in other embodiments the first and second molecular layers may have a different composition and/or structure.

Any appropriate stacking between the adjacent molecular layers may be used. For example, in some embodiments the molecular layers may be AB-stacked. In an AB-stacked graphene bilayer the planar layers are parallel and orientated relative to one another such that within either of the layers, three of the carbon atoms forming part of a hexagonal group of six carbon atoms are located directly above (or below) carbon atoms of the other layer. Furthermore, the other three carbon atoms of the six carbon atoms which form the hexagonal group are located directly above or below the central 'empty' spaces defined by a hexagonal group of six carbon atoms in the other layer.

In other embodiments the molecular layers may be AA-stacked. In an AA-stacked graphene bilayer the planar layers are parallel and orientated relative to one another such that within either of the layers, all of the carbon atoms forming a hexagonal group of six carbon atoms are located directly above (or below) carbon atoms of the other layer.

In other embodiments the molecular layers may be a mixture of AA-stacked and AB-stacked bilayers.

The covalent bonding between the first electrode and the second electrode may be any appropriate type of covalent bonding.

In the described embodiments the first molecular layer is covalently bonded to the second molecular layer in a manner such that the covalent bonding between the first and second molecular layers defines an aperture through both the first and second molecular layers. The aperture may be created in any appropriate way provided that it is defined by covalent bonding between the first and second molecular layers.

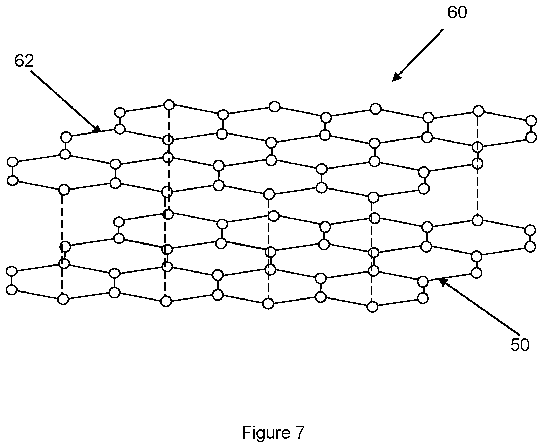

Figure 7 shows a multilayer structure. In this case the multilayer structure 60 is a bilayer graphene structure. That is to say that the multilayer structure 60 has two graphene monolayers (one atom thick layers) stacked on top of each other. The graphene monolayer shown in Figure 3 and indicated by 50 is a first graphene monolayer (also referred to as a first layer) of the bilayer graphene structure 60. A second graphene monolayer 62 (also referred to as a second layer) is stacked on the first graphene monolayer 50.

The first and second graphene monolayers can be said to be stacked in an AA configuration (or AA-stacked). A graphene bilayer which is AA stacked may be referred to as an AA stacked graphene bilayer or an AA graphene bilayer. It can be seen that in an AA stacked graphene bilayer the planar layers are parallel and orientated relative to one another such that within either of the layers, each carbon atom is located directly above (or below) a carbon atom of the other layer. In this context, directly above or below means located in a direction from the layer which is perpendicular to the plane of the layer.

The first and second molecular layers 10, 20 are weakly bonded adjacent to one another by van der Waals forces.

In order to produce the desired aperture defined by covalent bonding between the adjacent first and second molecular layers, the structure of the stacked first and second molecular layers must be measured before the aperture can be created. The measurement of the structure of the stacked planar molecular layers of the multilayer structure may be carried out using any appropriate measuring device such as, for example, a scanning tunnelling microscopy (STM) device. Methods of measuring the structure of molecular layers are well known. As such, further discussion of the process is omitted.

Once the structure of stacked first and second molecular layers has been measured, the stacked first and second molecular layers may be arranged in a desired orientation relative to a cutter (such as, for example, an STM lithography device) such that the cutter can be operated so as to simultaneously break bonds within the first and second molecular layers in order to produce edges in both the first and second molecular layers of a desired configuration which corresponds to the desired shape of aperture to be formed. The edges of the first and second molecular layers are then allowed to relax into an energetically more favourable state such that they covalently bond to one another to form the aperture through both the first and second molecular layers.

The cutting process performed by the cutter (i.e., the breaking of bonds in the molecular layers) may occur in an inert atmosphere or in vacuum so that the edge (created by the cutter breaking bonds in the molecular layers) does not bond with an atom with which it is not supposed to bond - i.e., by preventing the edges of two adjacent molecular layers which have been cut using the cutter from bonding with stray atoms, this ensures that the edges of the adjacent molecular layers can relax so that they covalently bond with one another as desired. In other words, by cutting the molecular layers in an inert atmosphere or vacuum, this prevents any cut edges from chemically reacting with the surrounding atmosphere.

In some embodiments of the invention it is not necessary to measure the structure of each of the first and second adjacent molecular layers individually. That is to say, in some embodiments, by measuring the structure of the first generally planar molecular layer, it is possible to infer the structure of the second adjacent generally planar molecular layer. For example, in the case where the multilayer structure is a bilayer graphene structure, the first and second generally planar molecular layers may have a known relative orientation in that they may be AA-stacked. Due to the fact that the first and second adjacent generally planar molecular layers in the bilayer graphene structure have a known relative orientation (i.e., are AA-stacked), then by measuring the structure of the first molecular layer it is possible to infer the structure of the second adjacent generally planar molecular layer.

Although in the embodiment described above the structure of the first generally planar molecular layer is measured and the structure of the second adjacent generally planar molecular layer is inferred from the measurement of the structure of the first molecular layer, this need not be the case in all embodiments of the present invention. For example, in some embodiments, the structure of the first and second adjacent generally planar molecular layers may be measured individually.

In some embodiments of the invention, once the structure of the first and second adjacent generally planar molecular layers has been determined, the multilayer structure may be arranged in the desired orientation relative to the cutter so that the cutter can break the bonds within the first molecular layer to produce the first edge of a desired configuration and then subsequently the multilayer structure may be arranged in a desired orientation relative to the cutter such that the cutter can be used to break bonds within the second molecular layer so as to produce a second edge of the desired configuration.

Arranging the multilayer structure in a desired orientation relative to the cutter and subsequently using the cutter to break bonds may also be referred to as cutting the multilayer structure (or layers of the multilayer structure) along a particular crystallographic direction.

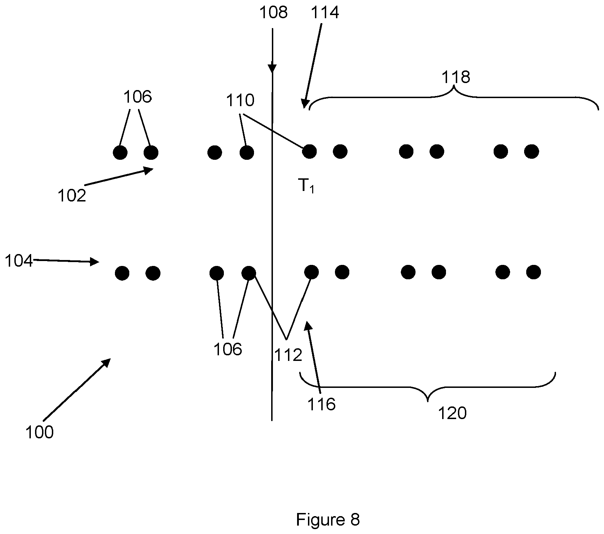

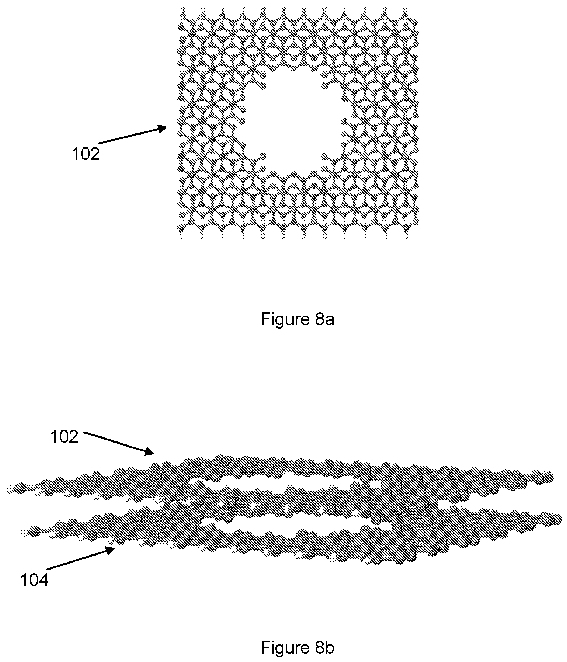

Figure 8 shows a schematic view of a portion of a multilayer structure 100 being cut by a cutter. In this case the multilayer structure 100 is a bilayer graphene (i.e. it consists of first and second adjacent molecular layers of graphene). The multilayer structure 100 has a first generally planar molecular layer 102 and a second generally planar molecular layer 104. The first layer 102 and second layer 104 are adjacent one another. As previously discussed, each of the generally planar molecular layers 102, 104 consists of an array of covalently bonded carbon atoms 106. The first layer 102 and second layer 104 are AA-stacked relative to one another and hence the first and second layers 102, 104 have a known relative orientation.

The multilayer structure 100 has been arranged in a desired orientation relative to a cutter. The cutter acts along a cutting axis 108. When the cutter is operated the cutter breaks bonds between adjacent atoms through which the cutting axis 108 passes. In this case, the multilayer structure 100 is arranged in a desired orientation relative to the cutter such that the cutting axis 108 passes between a first pair of bonded atoms 110 of the first layer 102 and a second pair of bonded atoms 112 of the second layer 104.

The cutter breaks bonds within the first generally planar molecular layer 102 to produce a first edge 114 of a desired configuration (in this case a $T_1$ configuration) corresponding to a desired configuration of aperture to be formed. The cutter also simultaneously breaks the bond between the atoms 112 within the second generally planar molecular layer 104 to produce a second edge 116 of a desired configuration (in this case also a $T_1$ configuration) corresponding to the desired shape of aperture to be formed. In this case the portion of the multilayer structure (stacked first and second molecular layers) which defines the desired configuration of aperture is portion 118 of the first layer 102 and portion 120 of the second layer 104. The first edge 114 of the first generally planar molecular layer 102 and the second edge 116 of the second generally planar molecular layer are allowed to relax so that the first edge 114 of the first generally planar molecular layer 102 and the second edge 116 of the second generally planar molecular layer 104 covalently bond to one another to define part of a desired configuration of aperture through both the first and second molecular layers.

The portions of the first and second molecular layers 102, 104 which are not portions 118 and 120 are discarded.

In the embodiment discussed above the cutting is done using Transmission Electron Microscopy (TEM) (discussed in, for example, S. Liu, Q. Zhao, J. Xu, K. Yan, H. Peng, F. Yang, et al., "Fast and controllable fabrication of suspended graphene nanopore devices," Nanotechnology, vol. 23, pp. 085301-085301, March 2 2012). In other embodiments any other appropriate cutting method may be used, such as those described in the following papers:

C. J. Russo and J. A. Golovchenko, "Atom-by-atom nucleation and growth of graphene nanopores," Proceedings of the National Academy of Sciences of the United States of America, vol. 109, pp. 5953-5957, Apr 17 2012.

A. T. Kuan and J. A. Golovchenko, "Nanometer-thin solid-state nanopores by cold ion beam sculpting," Applied Physics Letters, vol. 100, May 21 2012.

The generally circular apertures 84, 84a shown in Figures 5 and 6, which have a diameter of about 1.5nm, may be formed from AB-stacked bilayer graphene as follows. The multilayer structure (in this case AB-stacked bilayer graphene) is arranged in a desired orientation relative to a cutter. The cutter is operated to break bonds between adjacent atoms in each layer of the multilayer structure through which a cutting axis (as indicated by 108 in Figure 8) of the cutter passes. Figures 8a and 8b show a plan view and a perspective view respectively of a portion of AB-stacked bilayer graphene which has been cut by a cutter in order to produce an aperture 84, 84a as shown in Figures 5 and 6. Within figure 8a only the first molecular layer 102 of the multilayer structure is visible, whereas in Figure 8b both the first layer 102 and the second layer 104 of the multilayer structure are visible. The cutter and multilayer structure are orientated relative to one another, and the cutter is operated so as to break bonds between adjacent atoms in each layer of the multilayer structure and so as to remove atoms from each layer of the multilayer structure such that both the first layer 102 and second layer 104 of the multilayer structure have the structure of the first molecular layer 102 shown in figure 8a. The first and second molecular layers are then allowed to relax so as to covalently bond to one another to define the aperture 84, 84a through both the first and second molecular layers such as that shown in Figures 5 and 6.

The bonds within the first and second molecular layers which must be cut (for example, those shown in figures 8a and 8b) and allowed to relax so as to covalently bond to form first and second covalently bonded molecular layers which define a desired configuration of aperture through both the first and second molecular layers were determined as follows.

In order to predict the shape and size of an aperture which is formed by cutting first and second molecular layers and allowing them to covalently bond to one another, the relaxation first and second molecular layers of the multilayer structure whereby, atoms of one layer covalently bond with atoms of the adjacent layer, can be modelled. For example, the relaxation may be modelled using density functional theory (DFT). For example, the relaxation may be modelled using the SIESTA implementation of DFT. SIESTA (Spanish Initiative for Electronic Simulations with Thousands of Atoms) is a well-known method and software implementation for performing electronic structure calculations and ab initio molecular dynamics simulations of molecules and solids.

Using the DFT code of SIESTA, structural optimisation (e.g. to predict the shape of a aperture which is formed according to the present invention by cutting a particular shape out of a multilayer structure having first and second molecular layers) may be performed using the generalised gradient approximation (GGA) with Perdew-Burke-Ernzerhof (PBE) parameterization and double zeta polarized (DZP) basis sets of pseudo atomic orbitals for a plane wave cut-off energy of 250 Ry with maximum force tolerance of 40 meV/Å. k-point sampling of the Brillion zone was performed by $1 \times 1 \times 1$ Monkhorst-Pack grid. Where appropriate, simulations may also be carried out using periodic boundary conditions.

The use of the previously described embodiments of the invention as a sensor arrangement is now discussed.

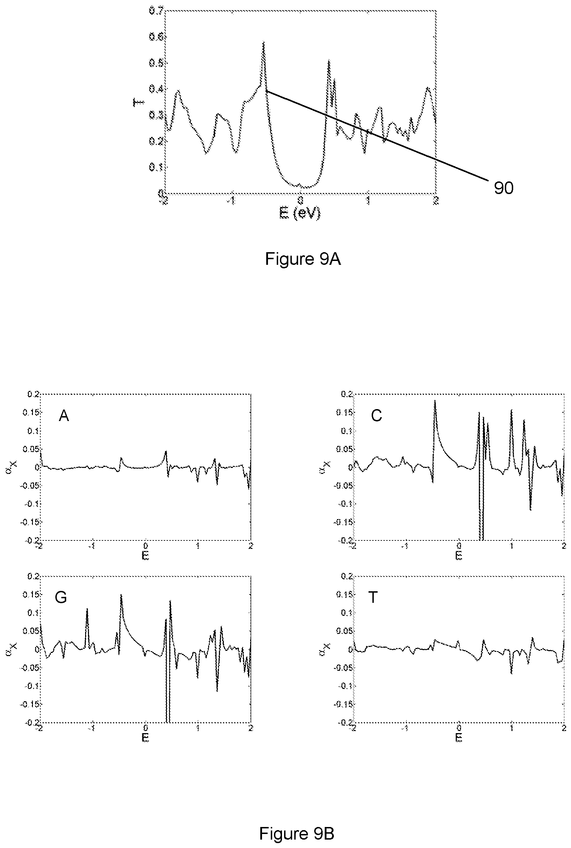

Figure 9A shows the transmission spectrum for the electrical sensor assembly shown in Figure 5. The transmission spectrum is a plot of transmission coefficient (T) against energy (E) in electron volts (eV). The energy (E) is the energy of the charge carriers (in this case electrons) within the electrical sensor assembly. The transmission spectrum of the electrical sensor assembly of Figure 5 shown in Figure 9A is indicated by line 90. In Figure 9A the transmission coefficients which form the transmission structure have been calculated for the flow of charge from the electrode 74 on the right of Figure 5 to the electrode 72 on the left of Figure 5.

The transmission spectrum of the electrical sensor assembly shown in Figure 5 (and the other transmission spectra within this document are calculated as follows.

First, the Hamiltonian of the system is calculated. The converged profile of charge via the self-consistent DFT loop for the density matrix implemented by SIESTA is used to obtain this Hamiltonian. Then the SMEAGOL method (which is well known in the art and which is described in detail in A. R. Rocha, V. M. Garcia-Suarez, S. W. Bailey, C. J. Lambert, J. Ferrer, and S. Sanvito, "Towards molecular spintronics," Nat Mater, vol. 4, pp. 335- 339, 2005) to calculate the Transmission coefficient T as follows:

$$T(E) = tr\{\Gamma_R(E)G^R(E)\Gamma_L(E)G^{R\dagger}(E)\} \quad (1)$$

where $\Gamma_{L,R}(E)$ are level broadening due to the coupling between left and right electrodes and scatter, and are given by:

$$\Gamma_{L,R}(E) = i(\Sigma_{L,R}(E) - \Sigma_{L,R}^{\dagger}(E)) \quad (2)$$

and where $\Sigma_{L,R}(E)$ are the retarded self-energies of the left and right leads.

$G^R(E)$ is the retarded Green's function of the system and is given by:

$$G^R = (ES - H - \Sigma_L - \Sigma_R)^{-1} \quad (3)$$

where H is the Hamiltonian matrix (obtained from the DFT self-consistent loop implemented by SIESTA) and S is the overlap matrix.

Landauer's formula can be used to determine the low-bias conductance G of the system at finite temperature T as a function of the transmission coefficient T(E) as follows:

$$G = G_0 \int_{-\infty}^{+\infty} dE T(E) \left(-\frac{\partial f}{\partial E}\right) \quad (4)$$

where $G_0$ is the conductance quantum given by:

$$G_0 = 2e^2/h \quad (5)$$

where e is the elementary charge and h is Planck's constant and where the Fermi-Dirac distribution function $f(E)$ is given by:

$$f(E) = (1 + \exp((E - \mu)/k_B T))^{-1} \quad (6)$$

where $\mu$ is the electrochemical potential, which is the same for both reservoirs at small bias voltage and $k_B$ is the Boltzmann constant.

Figure 9B shows a plot of the differential transmission spectrum $\alpha_x$ of the electrical sensor arrangement shown in Figure 5 when a particular portion of a chemical species X is passing through the aperture 84. In this case the portions of the chemical species X are the four different nucleotides of DNA: A (adenine) indicated by 92, G (guanine) indicated by 94, T (thymine) indicated by 96 and C (cytosine) indicated by 98.

The differential transmission spectrum $\alpha_x$ for chemical species (or portion of chemical species) X is given by:

$$\alpha_X(E) = \log_{10}(T_X(E)) - \log_{10}(T_0(E)) \quad (7)$$

where $T_x$ is the transmission spectrum when chemical species (or portion of chemical species) X is received by the aperture of the electrical sensor assembly and $T_0$ is the transmission spectrum when no chemical species is received by the aperture of the electrical sensor assembly (i.e. when the aperture is empty).

It can be seen from Figure 9b that the differential transmission spectra for each of the nucleotides are distinct. According to the invention the electrical sensor assembly shown in Figure 5 forms part of a sensor arrangement which includes an electrical power supply connected to the first and second electrodes. Whilst a chemical species (or portion thereof) is passed through the aperture 84 of the electrical sensor assembly 70, a voltage can be applied across the first and second electrodes and the current flowing between the first and second electrodes can be measured. The measured current flowing between the first and second electrodes is a function of both the voltage applied across the first and second electrodes and the transmission spectrum of the electrical sensor assembly when a particular chemical species (or part of a chemical species) is received by the aperture in the electrical sensor assembly.

The transmission spectrum T(E) can be obtained by measuring the current I(V) versus the source-drain voltage V in an asymmetric device, in which the resistance to current flow in the left electrode is substantially greater than that of the right electrode or vice versa. As examples, though not exclusively, this asymmetry can be achieved by making the right electrode wider than that of the left electrode, by making the right electrode from a different material than the left electrode. In such a device, the transmission coefficient T(E) at E=eV (where e is the electronic charge) is given by

$$T(E) = \frac{h}{2e^2}\frac{dI(V)}{dV} \quad (8)$$

Where dI(V)/dV is the differential conductance of the device at voltage V.

The fact that the electrical sensor assembly has a different transmission spectrum (and hence measured current for a given applied voltage) depending on what chemical species (or portion of chemical species) is received by the aperture enables identification of different chemical species because a particular chemical species passing through the aperture of the electrical sensor assembly results in a particular measured current between the first and second electrodes for a given applied voltage. Consequently, determining the current flow between the first and second electrodes when a particular voltage is applied across the first and second electrodes may enable identification of a particular chemical species (or part of a chemical species) which is received by the aperture of the electrical sensor assembly.

In certain situations, the transmission coefficient of an electrical sensor assembly at a particular energy (and hence applied voltage across the first and second electrodes of the electrical sensor assembly) may be substantially similar for several different chemical species (or parts of chemical species) when they are received by the aperture of the electrical sensor assembly. In such situations, it may be desirable to measure the current flow between the first and second electrodes not just for a single voltage, but for a plurality of voltages.

The current may be measured for any appropriate number of applied voltages, for example two or more. As discussed above, increasing the applied voltage corresponds to increasing the energy of the charge carriers (electrons) that flow through the electrical sensor assembly.

In some embodiments the applied voltage across the first and second electrodes may be 'swept'. Sweeping the voltage means applying a continually varying voltage across the first and second electrodes. For example, a saw-tooth voltage waveform may be used where the applied voltage increases from a negative voltage $-V_1$ to a positive voltage $+V_2$ via 0 at a constant rate in a time period t. After the voltage in any given period reaches $+V_2$, that period is ended and the next period is commenced, hence the voltage returns to $-V_1$ and the process repeats. Sweeping the voltage in this way results in a measured current which corresponds to the transmission spectrum between the energy which corresponds to $-V_1$ and the energy which corresponds to $+V_2$.

It will be appreciated that any appropriate changing voltage signal may be applied across the first and second electrodes - the maximum and minimum voltages may be any appropriate voltage, he maximum and minimum voltages may be the same polarity or different polarities, the shape of the voltage waveform may be any appropriate shape and the period of the voltage waveform may be any appropriate period.

As previously discussed, by applying several different voltages across the first and second electrodes (or applying a 'sweep' of voltages) this applies several different amounts of energy (or a 'sweeping' amount of energy) to the charge carriers within the electrical sensor assembly. Therefore the measured current flow between the first and second electrodes whilst applying the different voltages varies as a function of the transmission spectrum of the electrical sensor assembly and chemical species (or portion of chemical species) received within the aperture of the electrical sensor assembly at the time the current flow is measured.

It follows from the above that measuring the current flow between the first and second electrodes of an electrical sensor assembly whilst applying a several different voltages (or sweeping voltage) across the first and second electrodes enables a measure of the transmission spectrum of the electrical sensor assembly and whatever species (or portion of species) is received by the aperture in the electrical sensor assembly when the current is measured to be determined. This enables a characteristic transmission spectrum for the electrical sensor assembly and each chemical species (or portion of a chemical species) which may be received by the aperture of the electrical sensor assembly to be determined. If desired, the transmission spectrum of the electrical sensor assembly when no chemical species is located in the aperture may be subtracted from the combined transmission spectrum for the electrical sensor assembly and any chemical species (or part of a chemical species) received by the aperture in order to give a measure of the transmission spectrum of said chemical species (or part of a chemical species) which is more representative of the actual transmission spectrum of said chemical species (or part of a chemical species). An example of a measure of the transmission spectrum in which the transmission spectrum of the electrical sensor assembly when no chemical species is received by the aperture is subtracted from the transmission spectrum of the combined electrical sensor assembly and chemical species received by the aperture is the previously referred to differential transmission spectrum.

It follows that if the transmission spectrum of a particular chemical species (or portion thereof) is measured, then this transmission spectrum can be compared with previously determined transmission spectra for various chemical species (or portions thereof) in order to determine a match and hence identify the chemical species (or portion thereof) which is currently received by the aperture of the electrical sensor assembly.

In more detail, in some embodiments, before the electrical sensor assembly can be used it may undergo some calibration. For example, in order to determine the differential transmission spectrum (or differential transmission coefficient) it is necessary to determine the transmission spectrum (or transmission coefficient) at the applied voltages (or voltage) at which measurements are to be carried out by the electrical sensor assembly. As such, the transmission spectrum (or transmission coefficient) at the applied voltages (or voltage) are measured in the absence of any chemical species in the aperture. This information may be stored by any appropriate method, for example in a memory. The information can then be accessed at a later point in order to determine, if required, the differential transmission spectrum (or differential transmission coefficient).

Furthermore, if the electrical sensor assembly is to be used to identify a particular, target chemical species (or portion of a chemical species) then the calibration process may include inserting the target chemical species (or portion thereof) into the aperture of the electrical sensor assembly and measuring an electrical property (e.g. transmission coefficient, transmission spectrum, differential transmission coefficient, or differential transmission spectrum) whilst the target chemical species (or portion thereof) is received by the aperture. The measured electrical property can then be stored by any appropriate method, for example a memory. When the electrical sensor assembly is used, the same electrical property that was measured to arrive at the stored electrical property can be measured when other chemical species (or portions thereof) are passed through the aperture. The measured electrical property may be supplied to a processor. The processor is configured to compare the measured electrical property with the stored electrical property. When the processor determines the measured electrical property is within a predetermined range of the stored electrical property then the processor may produce an output that indicates that the target chemical species (or portion of a chemical species) has been received by the aperture of the electrical sensor assembly.

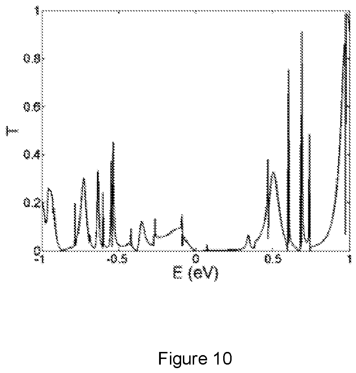

Figure 10 shows the transmission spectra for the electrical sensor assembly shown in Figure 6. The transmission coefficient applies for movement of charge carriers (electrons) in the direction from the second molecular layer 78a to the first molecular layer 76a (i.e. from the second electrode 74a to the first electrode 72a).

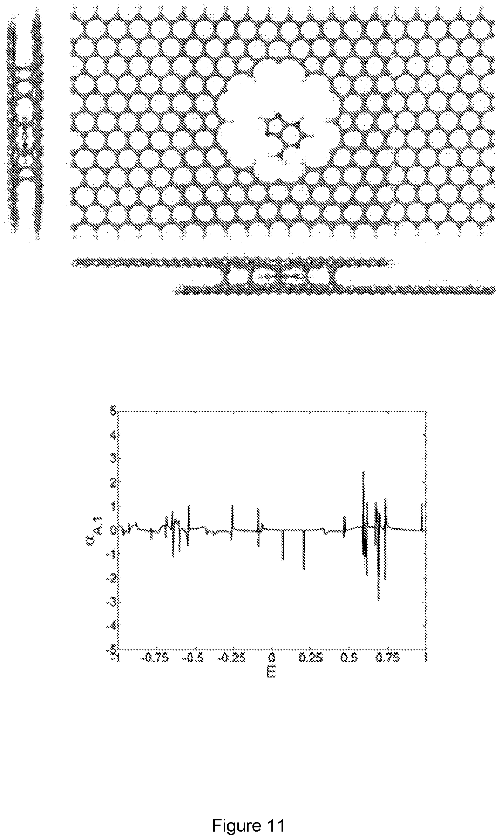

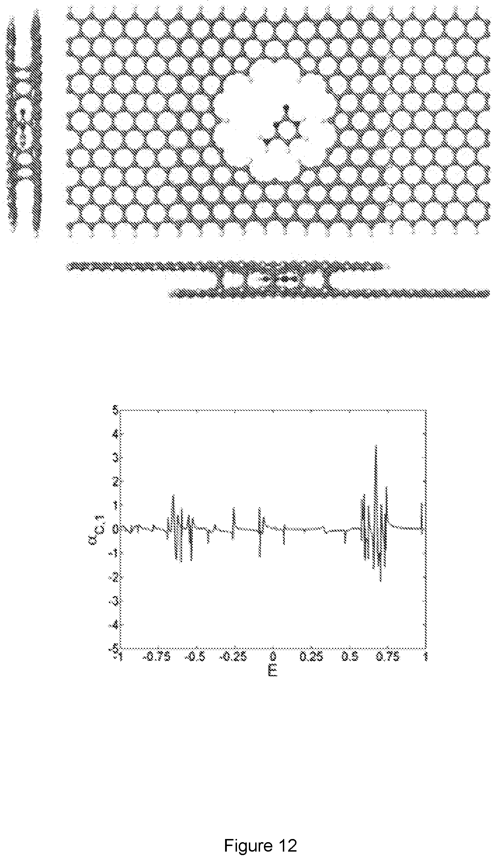

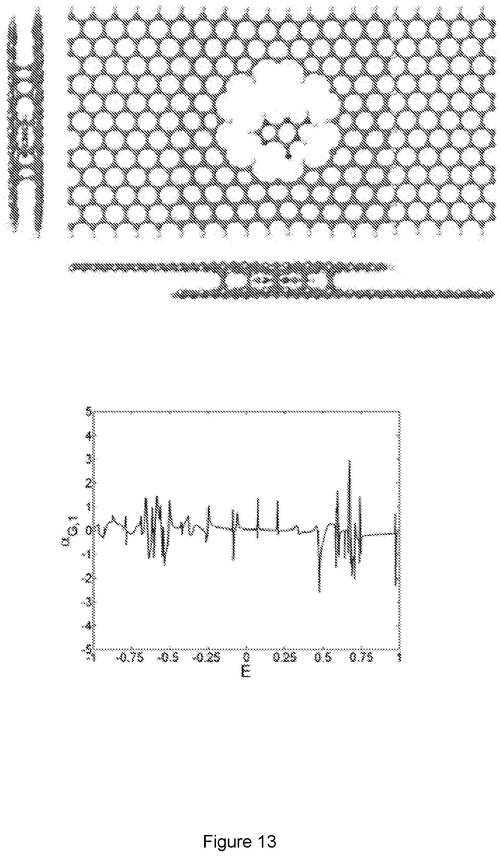

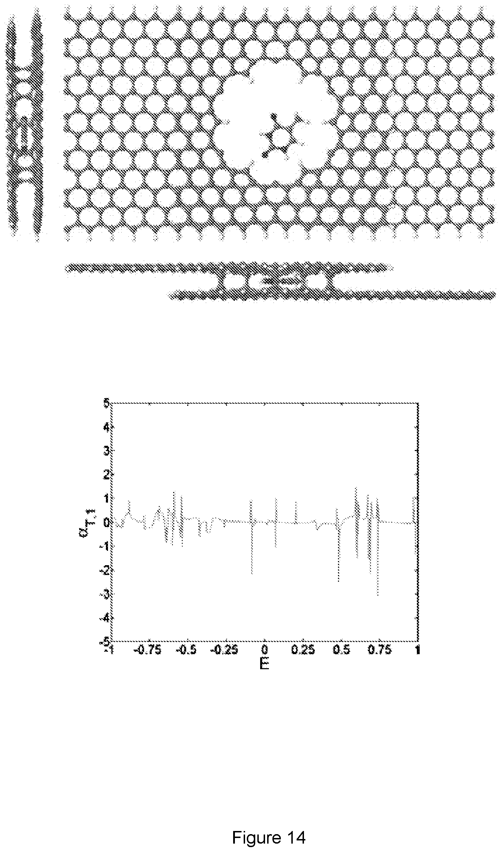

Figures 11 to 14 each show both schematic views of the electrical sensor assembly shown in Figure 6 whilst a particular nucleobase of a nucleotide of DNA is located within the aperture of the electrical sensor assembly, and an associated graph. In Figure 11 the nucleobase is adenine; in Figure 12 the nucleobase is cytosine; in Figure 12 the nucleobase is guanine; and finally, in Figure 14 the nucleobase is thymine.

The graphs in each of Figures 11 to 14 show differential transmission spectra for the electrical sensor assembly when each of the respective nucleobases is received by the electrical sensor assembly.

Again, it can be seen that the differential transmission spectra of each nucleobase is unique. Consequently, as previously discussed in relation to the electrical sensor assembly shown in Figure 5, it is possible to distinguish between each of the nucleobases of a nucleotide of DNA by applying a voltage (either a constant voltage, several different voltages, or a sweeping voltage) across the first and second electrodes of the electrical sensor assembly and measuring the current flow between the first and second electrodes.

Furthermore, the electrical sensor assembly may be calibrated by passing a strand of DNA with a known sequence of nucleotides through the aperture of the electrical sensor assembly. An electrical property (e.g. transmission coefficient, transmission spectrum, differential transmission coefficient, or differential transmission spectrum) of each nucleotide can be determined from the applied voltage and measured current as the DNA strand passes through the aperture. Because the sequence of nucleotides within the DNA strand is known, the determined electrical property of each nucleotide can be stored as an electrical property which is indicative of the known type of nucleotide. In this way, an electrical property for each type of nucleotide can be stored, for example, in a memory. Subsequently, a strand of DNA with an unknown sequence of nucleotides can be passed through the aperture of the electrical sensor assembly. The electrical property of each nucleotide passing through the aperture is measured and supplied to a processor. The processor is configured to compare the measured electrical property of each nucleotide with the stored electrical property for each type of nucleotide. The processor can then determine which stored electrical property the measured electrical property for each nucleotide is closest to and thereby determine the identity of each nucleotide within the strand of DNA. The processor may then output a signal which is indicative of the determined sequence of nucleotides within the strand of DNA which has passed through the aperture of the electrical sensor assembly.

It will be appreciated that the differential transmission spectra shown in Figures 11 to 14 are when the relevant nucleobase is in a particular orientation relative to the electrical sensor assembly (i.e. the particular orientation of the nucleobase relative to the electrical sensor assembly shown in the relevant figure). It was thought by the applicant that the transmission spectra (and hence differential transmission spectra) of the electrical sensor assembly when the aperture has received a particular nucleobase of a nucleotide may vary slightly depending upon the orientation of the nucleobase of a nucleotide relative to the electrical sensor assembly. In order to assess this, the average differential transmission spectra $\overline{\alpha_x}$ for each nucleobase X were calculated according to the formula:

$$\overline{\alpha}_X(E) = \frac{1}{m_{\max}} \sum_{m=1}^{m_{\max}} \alpha_{X,m}(E) \qquad (9)$$

where $\alpha_{X,m}$ is the mth differential transmission spectrum for nucleobase X and there are $m_{\max}$ differential transmission spectra for nucleobase X in total. The probability distribution $P_x$ is therefore obtained by sampling all such orientations.

The probability distribution of the set$\{\alpha_{X,m}(E)\}$ for energies E greater than a minimum energy $E_{\min}$ and less than a maximum energy $E_{\max}$, for configurations m from m=1 to m=$m_{\max}$, where $m_{\max}$ is the total number of configurations, and for a given chemical species X (e.g. nucleobase), is defined as:

$$P_X(\alpha) = \frac{1}{m_{max}(E_{max} - E_{min})} \sum_{m=1}^{m_{max}} \int_{E_{min}}^{E_{max}} dE \delta(\alpha - \alpha_{X,m}(E)) \qquad (10)$$

where, based on equation 7, the quantity $\alpha_{X,m}(E)$ is defined, for a configuration m and energy E, as:

$$\alpha_{X,m}(E) = \log_{10}(T_{X,m}(E)) - \log_{10}(T_0(E)) \qquad (11)$$

where $T_{X,m}$ is the transmission spectrum when chemical species (or portion of chemical species) X in configuration m is received by the aperture of the electrical sensor assembly, and $T_0$ is the transmission spectrum when no chemical species is received by the aperture of the electrical sensor assembly (i.e. when the aperture is empty).

The relationship in equation 11 has the property that the number of values of $\alpha_{X,m}(E)$ between $\alpha$=a and $\alpha$=b is:

$$\int_a^b P_X(\alpha) \qquad (12)$$

because:

$$\int_a^b dx \, \delta(x - x_0) = 1$$

only when $a < x_0 < b$

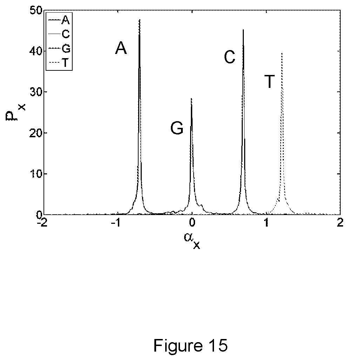

In this example, to obtain $P_x$ shown in figure 15, the applicant calculated the differential transmission spectra for each of the nucleobases (A, C, G, T) using four different differential transmission spectra, each corresponding to a different orientation of nucleobase relative to the electrical sensor assembly (also referred to as a different configuration m). The four orientations used four each differential transmission spectrum were i) the orientation of the nucleobase relative to the electrical sensor assembly shown in each of the figures 11 to 14, ii) the orientation of the nucleobase relative to the electrical sensor assembly shown in each of the figures 11 to 14, but with the relevant nucleobase rotated relative to the electrical sensor arrangement in the plane of the figure by 90° clockwise, iii) the orientation of the nucleobase relative to the electrical sensor assembly shown in each of the figures 11 to 14, but with the relevant nucleobase rotated relative to the electrical sensor arrangement in the plane of the figure by 180° clockwise, and iv) the orientation of the nucleobase relative to the electrical sensor assembly shown in each of the figures 11 to 14, but with the relevant nucleobase tilted by 30° relative to the plane of the electrical sensor arrangement.

Figure 15 shows the resulting plots of the distribution $P_x$ for each of the nucleobases being received by the aperture of the electrical sensor assembly shown in Figure 6. It can be seen that the distributions $P_x$ are sufficiently different such that it is possible to differentiate between the differential spectra and hence between the different nucleobases to thereby determine which nucleotide is received by the aperture of the electrical sensor assembly at any given time.

Any appropriate signal processing method may be used to discriminate between different chemical species (or portions thereof). For example, in an alternative method, a sensor arrangement including an electrical sensor assembly may be configured to determine a measure of the transmission coefficient $T(E)$ of the electrical sensor assembly in the presence of each of the chemical species to be detected- in this example, each of the four bases $X=[A, C, G, T]$. The transmission coefficient depends on the orientation of the base within the aperture. In one example, for each base $X$, a number ($m_{max}$) of distinct orientations of a base within the aperture (labelled $m=1, ... m_{max}$) is considered. The resulting transmission coefficients are denoted $T_{X,m}(E)$. As an alternative method of achieving the required selectivity between each of the bases, the quantity $\beta_{X,m}$ may be measured, which can be measured using any two-electrode geometry, such as those shown in figures 5 and 6:

$$\beta_{X,m}\left(V\right) = \log_{10}\left(I_{X,m}\left(V\right)\right) - \log_{10}\left(I_0\left(V\right)\right) \qquad (13)$$

where $I_{X,m}(V)$ is the current through the device at voltage $V$, in the presence of nucleobase $X$, with orientation m, defined by:

$$I_{X,m}\left(V\right) = \frac{2e}{h}\int_{E_F - \frac{eV}{2}}^{E_F + \frac{eV}{2}} dE\, T_{X,m}(E) \qquad (14)$$

In equation 13, the quantity $I_0(V)$ is the current through the 'bare' device in the absence of any nucleobase.

The probability distribution of the set $\{\beta_{X,m}(E)\}$ for a given base X is then defined by

$$P_X\left(\beta\right) = \frac{1}{m_{max}\left(eV_{max} - eV_{min}\right)} \sum_{n=1}^{m_{max}} \int_{eV_{min}}^{eV_{max}} dV\, \delta(\beta - \beta_{X,m}(V)) \qquad (15)$$

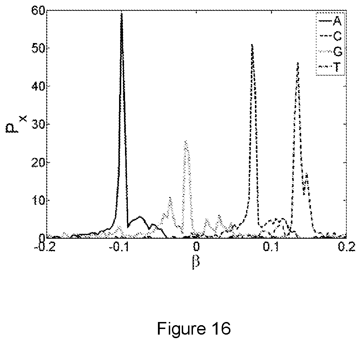

Figure 16 shows a plot of the probability distribution $P_X(\beta)$ of the set $\{\beta_{X,m}(E)\}$ for each of the nucleobases A, C, G and T. The probability distribution $P_X(\beta)$ was obtained using an electrical sensor assembly having a similar structure to that shown in Figure 6, but fabricated from silicene as opposed to graphene.

Figure 16 shows that the quantity $P_X(\beta)$ can be used to distinguish between bases in the nanopore. The presence of well-separated peaks demonstrates that through this alternative signal processing method, the bases can be selectively detected. The heights and positions (or locations) of the peaks are different for a given base and either of them could be used to select and recognize the base type. For example, a sensor arrangement according to the present invention may include an electrical sensor assembly as described above and a processor configured to measure the probability distribution $P_X(\beta)$ and analyse the location of a peak in the probability distribution $P_X(\beta)$ and/or the height of a peak in the probability distribution $P_X(\beta)$ so as to recognise the probability distribution $P_X(\beta)$ of a particular base passing through the aperture of the electrical sensor assembly, and hence identify which particular base is passing through the aperture of the electrical sensor assembly. It follows that an electrical sensor assembly according to the present invention (for example, a nanopore formed of silicene) may be used for DNA sequencing.

Although the method above has been described in relation to the use of an electrical sensor assembly formed from silicene to identify bases of DNA, it will be appreciated that in other embodiments of the invention the electrical sensor assembly may be formed from any appropriate first and second generally planar molecular layers each consisting of an array of covalently bonded atoms, and the chemical species (or portion thereof) that it is desired to detect may be any appropriate chemical species (or portion thereof).

The description above shows that it is possible to distinguish between different chemical species which are received by the aperture of the electrical sensor assembly by applying a voltage across the electrodes of an electrical sensor assembly in accordance with the present invention and measuring the current between the electrodes. Although the chemical species which have previously been described are nucleobases of DNA, it will be appreciated that an electrical sensor assembly according to the present invention (or a sensor arrangement according to the present invention) can be used to measure any suitable electrical property of any suitable chemical species which may be received by the aperture of the electrical sensor assembly. It will be appreciated that, depending on the size, shape and/or configuration of the chemical species of which it is intended to measure a property, the electrical sensor assembly maybe configured such that the aperture of the electrical sensor assembly can receive the relevant chemical species.

Due to the fact that, as discussed above, it is possible to use a sensor arrangement or electrical sensor assembly according to the present invention in order to identify different nucleobases, a sensor arrangement or electrical sensor assembly according to the present invention can be used to identify nucleobases of the nucleotides within a strand of DNA and hence sequence the strand of DNA.

In order to use a sensor arrangement or electrical sensor assembly according to the present invention to sequence DNA, it is necessary to pass a strand of DNA through the aperture within the electrical sensor assembly such that the strand of DNA passes through the aperture of the electrical sensor assembly in a single direction of travel. In this way, as each nucleotide of the DNA strand passes through the aperture of the electrical sensor assembly in order, the nucleobase of each nucleotide can be identified by the sensor arrangement or electrical sensor according to the present invention and hence the sequence of the nucleotides within the DNA strand can be measured.

In order to cause a strand of DNA to pass through the aperture of an electrical sensor assembly according to the present invention it may be necessary to guide the strand of DNA to the aperture. Any appropriate method may be used to achieve this. One example is to have first and second chambers separated by a dividing wall which includes the electrical sensor assembly and hence the aperture thereof. The DNA strand is supported in a fluid in the first chamber. The fluid is urged from the first chamber into the second chamber (for example using one of the methods discussed below). Because the only way of fluid passing from the first chamber to the second chamber is via the aperture of the electrical sensor assembly in the dividing wall, the DNA strand is urged towards the aperture and then passes therethrough.

The dividing wall can be formed from any appropriate material. The dividing wall is substantially impermeable to the DNA strand and to the supporting fluid. In some embodiments the dividing wall may be formed from a generally planar molecular layer of electrically insulating material. One example of such a material is boron nitride, but any such material may be used. The first and second molecular layers of the electrical sensor assembly may be deposited on the molecular layer of insulating material. A cutter as previously described can then be used to cut the first and second molecular layers of the electrical sensor assembly to cause the aperture in the electrical sensor assembly to be formed whilst simultaneously creating an aperture in the layer of insulating material adjacent to the first and second molecular layers. The DNA strand and supporting fluid can thus pass through the aperture in the molecular layer of insulating material and through the adjacent aperture in the electrical sensor assembly. Due to the fact that the molecular layer on which the first and second molecular layers of the electrical sensor assembly are deposited is an insulating material, the molecular layer of insulating material does not interfere with the electrical signals of the electrical sensor assembly.

Any appropriate method may be used to cause a DNA strand to pass through the aperture of the electrical sensor assembly in a single direction of travel.

For example, the DNA strand and electrical sensor assembly may be located in a fluid and there may be a difference in pressure between the fluid on one side of the aperture of the electrical sensor assembly and the fluid in the other side of the aperture of the electrical sensor assembly such that fluid flow from the high pressure side to the low pressure side via the aperture causes the DNA strand to pass through the aperture. In another embodiment, the aperture of the electrical sensor assembly may be orientated such that the strand of DNA (and in some embodiments a fluid supporting the strand of DNA) passes through the aperture in the electrical sensor assembly by virtue of gravity or an electric field.

Alternatively the DNA may be supported in an ionic fluid. In this case, as is the case with known nanopore DNA sequencers previous discussed, electrodes may be placed either side of the aperture of the electrical sensor such that a voltage can be applied across the ionic fluid, thereby causing the ions within the ionic fluid to flow towards one of the electrodes, thereby moving the DNA strand through the aperture of the electrical sensor assembly.

It will be appreciated that any appropriate method may be used in order to cause the strand of DNA to move through the aperture within the electrical sensor assembly in a substantially single direction of travel.

Use of an electrical sensor assembly according to the present invention (and sensor arrangements comprising electrical sensor assemblies according to the present invention) for measuring a property of a chemical species, particularly in relation to measuring the properties of strands of DNA, is beneficial compared to known sensor assemblies used for DNA sequencing for several reasons.

First, as previously discussed, it is important that the nanopore (i.e. aperture) is stable. In particular, if the size or shape of the nanopore changes, this may have an adverse effect on any measurements taken. For example, in the case of known ionic flow methods, if the size of the nanopore changes, then this changes the rate at which ions can pass through the nanopore and hence leads to an incorrect current measurement from which it is not possible to correctly identify which nucleotide of a DNA strand is passing through the nanopore at any one time. Furthermore, in the case of electrical sensor assemblies according to the present invention, a change in the size or shape of the aperture results in the transmission spectrum of the electrical sensor assembly changing, again meaning that the characteristics of the system have changed such that it is no longer possible, without re-calibrating the system, to measure current flow in order to identify which nucleotide is received by the aperture within the electrical sensor assembly at any given time.

Producing the electrical sensor from a multilayer material such that a first molecular layer covalently bonds to a second molecular layer in order to define the aperture results in an aperture which has a very stable shape, size and configuration. This compares favourably with known nanopores which have been fabricated from single layer graphene, which can undergo self-shrinking (i.e. where the diameter of a hole cut in graphene may reduce because the layer of graphene finds it energetically favourable to do so) and abrasion of the edge of the nanopores due to the edges of the nanopore interacting with material passing through the nanopore.

Embodiments of the present invention may include an electrical sensor assembly which is formed from a semiconducting material. For example, graphene in bi-layer form (e.g. in embodiments which have first and second molecular layers which are both graphene) acts as a semiconductor. This contrasts to single layer graphene which acts generally as a conductor. The generally semiconducting nature of bi-layer graphene results in the electrical sensor being more sensitive to charge variation (i.e. charge located within the aperture) than single layer graphene. This additional sensitivity of semiconducting materials such as bi-layer graphene means that it would not be possible using a conducting material such as graphene to distinguish between nucleotides of a strand of DNA using a power supply and a current measuring device as discussed in relation to the present invention.

Within the previously described prior art DNA sequencing sensors, the use of ionic current has several disadvantages.

First, the known embodiments of DNA sequencing sensor require the ionic current to move the DNA strand through the nanopore. As previously discussed, the electrical sensor assembly according to the present invention can use any suitable method for moving the strand of DNA through the nanopore. For example, if either gravity or fluid pressure difference is used, then this may significantly reduce the complexity of the nanopore sequencing device.

Secondly, use of ionic current to move the DNA strand through the nanopore places practical limits on the speed at which the DNA strand can move through the nanopore. This is because prohibitively high voltage may be required in order to make the ions within the ionic fluid flow any faster.

In addition, the electronic response of the known nanopore DNA sequencing device is limited by the fact that the charge carriers within the system between the electrodes are ions within a liquid. The speed at which these charge carriers within the liquid can move is significantly less than the speed at which the charge carriers (electrons) within the electrical sensor assembly according to the present invention can move. This means that, because electrical signals of the known DNA sequencing device are slower than those of the present invention, that it is possible for the electrical sensor assembly of the present invention to operate at a faster speed than the known device, thereby resulting in increased throughput speed of the DNA strand through the electrical sensor assembly, hence reducing the time required to sequence a given length of DNA strand.

Finally, because prior art devices which utilise ionic current require electrodes on either side of the nanopore to generate an electric field which causes the ions to flow through the nanopore, the spaced electrodes and the ionic liquid between them act like a capacitor. The capacitance of the electrodes and liquid reduce the ability of the device to use alternating currents because the capacitance results in an RC time constant of the system which adversely affects non constant voltage signals which are applied to the device.

In addition to the disadvantages of known DNA sequencing devices discussed above which are overcome by DNA sequencing devices including an electrical sensor assembly according to the present invention, DNA sequencing devices including an electrical sensor assembly according to the present invention have several further advantages over known ionic sequencing methods. These advantages stem from the fact that in the known ionic sequencing devices the ionic current both causes the transport of the DNA strand through the nanopore, and is used to measure a property of each nucleotide as it passes through the nanopore to enable the nucleotides to be identified and therefore sequenced. To the contrary, using an electrical sensor assembly according to the present invention as part of a DNA sequencing device, means that a separate method can be used to transport the strand of DNA through the aperture (nanopore) of the electrical sensor assembly and to measure a property of each nucleotide as it passes through the nanopore. Because of this electrical sensor assemblies according to the present invention can be used to sequence DNA in ways that are not possible with the known ionic sequencing devices.

For example, it is possible to have a plurality of electrical sensor assemblies according to the present invention working in series or parallel with one another in a single sequencing vessel.

In more detail, a plurality of electrical sensor assemblies according to the present invention may be located as part of a dividing wall which separates a first portion of a sequencing vessel which contains a supporting fluid and a plurality of DNA strands to be sequenced, and a second portion of the sequencing vessel into which the DNA strands and supporting fluid pass via the respective apertures of the electrical sensor assemblies. Because voltage can be applied across each of the electrical sensor assemblies individually, and likewise the current flow in each electrical sensor assembly can be measured individually, it is possible to simultaneously sequence as many separate DNA strands as there are electrical sensor assemblies. Simultaneous sequencing of many separate DNA strands may be referred to as the plurality of electrical sensor assemblies working in parallel. The ability of multiple electrical sensor assemblies to work in parallel to sequence DNA may reduce the time required to sequence multiple DNA strands, increasing the throughput of a DNA sequencing device having such a construction.

Alternatively or in addition, two or more electrical sensor assemblies in accordance with the present invention may be arranged such that a DNA strand to be sequenced passes through the apertures of each of the electrical sensor assemblies in turn. As before, voltage can be applied across each of the electrical sensor assemblies individually, and likewise the current flow in each electrical sensor assembly can be measured individually. The separate measured current signals from each of electrical sensor assemblies may be compared with one another to produce a signal with improved integrity compared to the signals produced by the individual electrical sensor assemblies. Sequencing the same DNA strand with a plurality of electronic sensor assemblies may be referred to as the electronic sensor assemblies working in series. For example the first of two electrical sensor assemblies working in series may determine that the sequence of a strand of DNA is G, A, G, ?, C, A, T. The second of two electrical sensor assemblies working in series may determine that the sequence of a strand of DNA is G, A, G, A, C, A, ?. The question marks represent nucleotides which, for some reason, could not be identified by the respective electrical sensor assembly. A processor may compare the sequences determined by each electrical sensor assembly and determine that the correct sequence of the strand of DNA is G, A, G, A, C, A, T. In other embodiments any appropriate comparison of signals from electrical sensor assemblies in series may be utilised in order to produce a sequence with improved integrity.

A further benefit of an electrical sensor assembly according to the present invention is that the length of the aperture (in the direction of travel of the chemical species (e.g. DNA strand) through the aperture) can be selected so as to maximise the selectivity of the electrical sensor assembly and thereby maximise the sensitivity of the electrical sensor assembly to identify which chemical species (or portion of chemical species - e.g. nucleobase and hence nucleotide of a DNA strand) is received by the aperture in the electrical sensor assembly at any given time.

For example, it is known that the spacing between adjacent nucleobases on the strand of DNA is about 0.34 nanometres. It is advantageous for the length of the aperture (in the direction of travel of the DNA strand through the aperture) to be as close as possible to the separation distance between two adjacent nucleobases. This is so that that the largest possible portion of the DNA strand is located within the aperture of the electrical sensor assembly, thereby promoting the greatest interaction between the strand of DNA and the electrical sensor assembly, without the length of the aperture being so great as to contain more than one nucleobase which could result in the measured current flow between the first and second electrodes of the electrical sensor assembly being a function of the interaction of two different nucleobases with the electrical sensor assembly. If the measured current flow between the first and second electrodes of the electrical sensor assembly is a function of the interaction of two different nucleobases with the electrical sensor assembly, then this may adversely affect the ability of the electrical sensor assembly to be used to identify individual nucleobases.

In the case of DNA, in which the distance between adjacent nucleobases is about 0.34 nanometres, it has been found that bi-layer graphene is an ideal material for forming the electrical sensor assembly from because the spacing between the graphene layers is also about 0.34 nanometres. Consequently, the length of the aperture is also about 0.34 nanometres.

Although the previously discussed embodiments are used to measure a property of DNA, an electrical sensor assembly or sensor arrangement according to the present invention may be used to measure a property of any appropriate polymer. In order for the electrical sensor or sensor arrangement to distinguish between portions of the polymer as the length of the polymer moves through the aperture of the electrical sensor assembly, the polymer may have an inhomogeneous charge distribution along its length, like DNA. If, like DNA, the polymer includes spaced functional groups, then, as previously discussed, the electrical sensor assembly is most effective at discriminating between adjacent functional groups if the thickness of the aperture (and hence the spacing between the first and second molecular layers) is approximately the same as the spacing between adjacent functional groups. To this end, the material from which each of the first and second generally planar molecular layers is formed may be chosen such that a desired spacing between adjacent molecular layers is achieved. In such scenarios, the first and second molecular layers may be of the same material or of different materials. Likewise, if the chemical species of interest is a chemical species having a finite length, then the material(s) from which the electrical sensor assembly are formed may be chosen such that the length of the aperture corresponds to the length of the chemical species.

If it is desired to apply a plurality of voltages to a single chemical species (or portion of a chemical species) - for example applying a voltage sweep to a nucleotide - then it is desirable that the period of time during which the plurality of voltages is applied across the electrodes of the electronic sensor arrangement (e.g. the period of a voltage sweep) is less than or equal to the time that it takes for the chemical species (or portion of a chemical species - e.g. a nucleotide) to pass through the nanopore. This enables the current measuring device to measure current which is a function of the different applied voltages for each chemical species (or portion of a chemical species - e.g. a nucleotide).

The rate of passage of a nucleotide through the aperture of the electrical sensor assembly may be about 1 to about 5 μs. However, it will be appreciated that in other embodiments the strand of DNA may pass through the aperture of the electrical sensor assembly at any appropriate speed.

If a DNA strand moves through the aperture of the electrical sensor assembly at a rate of about 1 μs to about 5 μs per nucleobase/nucleotide, then if a periodic voltage signal such as a voltage sweep is to be applied across the electrical sensor assembly, the frequency of the sweeping voltage applied between the first and second electrodes may be between about 0.2 MHz and about 1 MHz. However, it will be appreciated that any appropriate frequency of periodic voltage signal may be used.

Although in the previously described embodiments the multilayer structure used to produce a molecular structure according to the present invention is a bilayer structure, any appropriate multilayer structure may be used. For example, the multilayer structure may have any appropriate number of adjacent generally planar molecular layers provided that this number is at least two.

In some embodiments of the invention, before the cutter is used to break bonds within the first and/or second molecular layer of the multilayer structure, the multilayer structure may be cooled to a temperature at which the relaxation of the first edge of the first molecular layer and the second edge of the second molecular layer (i.e., so that the first edge and second edge covalently bond to one another) may be substantially prevented. Once the cutter has broken a desired number of bonds within the first and/or second molecular layer of the multilayer structure, the multilayer structure may be subsequently heated to a temperature of between 100°C and 400°C at which the first edge of the first molecular layer and the second edge of the second molecular layer are permitted to relax so that the first edge and second edge can covalently bond to one another.

The previously described embodiments apply a voltage across the electrical sensor assembly and measure the current that flows between the electrodes in order to measure a property of the chemical species which is received by the aperture. Measuring the current that flows between the electrodes as a function of applied voltage enables a measure of the transmission coefficient of the electrical sensor assembly when the chemical species is received by the aperture of the electrical sensor assembly to be determined. This is an electrical property of both the electrical sensor assembly and the received chemical species which is a manifestation, in part, of how the electric structure of the chemical species interacts with the electric structure of the electrical sensor arrangement. A differential transmission coefficient of the electrical sensor assembly and the received chemical species can then be determined by a logarithm of the transmission coefficient when no chemical species is received by the aperture from the transmission coefficient when a chemical species is received by the aperture. This is another electrical property. It is intended to relate more closely to the electric structure of the chemical species received by the aperture than the transmission coefficient. Finally, either the transmission coefficient or the differential transmission coefficient may be used, after calibration, to identify what the chemical species received by the aperture is. Consequently, the electrical sensor assembly (and sensor arrangement) according to the present invention measure a physical property of the chemical species - i.e. what the identity of the chemical species is.

It will be appreciated that in other embodiments of the invention the electrical sensor assembly (or sensor arrangement) may measure any appropriate electrical property of the chemical species and may then use this information to determine any appropriate physical property of the chemical species.

A sensor arrangement according to the present invention may comprise processing means configured to receive electrical parameters of the electrical sensor assembly (e.g. applied voltage and measured current) and to then calculate a value indicative of an electrical property of the electrical sensor assembly and chemical species (if received by the aperture) - e.g. the transmission coefficient or differential transmission coefficient. The processing means may be configured to output the value indicative of an electrical property and/or to, based on the value indicative of an electrical property, determine a further property of the chemical species (if received by the aperture) - e.g. identify the chemical species - and provide an output based on said determination.

Within the described embodiments of electrical sensor assembly, the first and second electrodes both have the same shape and are both generally rectangular, with the aperture either being at the centre of the rectangular electrodes or at an end of the rectangular electrodes. In other embodiments the shape of the first and second electrodes may be different. In addition the electrodes may be of any appropriate shape. For example the electrodes may be generally triangular, with the aperture being located at one of the corners of the triangle, or the electrodes may have any other shape which increases in width with greater distance from the aperture. Such shapes help to minimise the electrical resistance in parallel to the aperture, thereby increasing the sensitivity of the aperture.

It will be appreciated that numerous modifications to the above described designs may be made without departing from the scope of the invention as defined in the appended claims.

**Claims**

1. A sensor arrangement comprising an electrical sensor assembly (70) suitable for measuring a property of a chemical species, the electrical sensor assembly comprising:

   first and second electrodes (72, 74) comprising first and second generally planar molecular layers (76, 78), the first and second generally planar molecular layers each consisting of an array of covalently bonded atoms (80); and
   an aperture (84) through both the first and second molecular layers; and
   wherein the aperture is configured to enable the chemical species to pass through the aperture; and
   the sensor arrangement further comprising an electrical power supply (86) connected to the first electrode and the second electrode, wherein the power supply is configured to apply a voltage across the first electrode and second electrode;
   **characterised in that**
   the first molecular layer is covalently bonded (82) to the second molecular layer to define said aperture; and
   the sensor arrangement further comprising a current measuring device (88) configured to measure current flow between the first and second electrodes.

2. A sensor arrangement according to 1, wherein the first electrode (72) comprises the first molecular layer (76) and the second electrode (74) comprises the second molecular layer (78), wherein the first electrode generally extends in a first direction away from the aperture (84) and away from the second electrode (74), and wherein the current flow between the first and second electrodes flows at least partially via the covalent bonding (82) between the first and second molecular layers.

3. A sensor arrangement according to any preceding claim, wherein
   the array of covalently bonded atoms (80) of one or both of the first and second generally planar molecular layers (76, 78) is a repeating structure, the repeating structure repeating in two substantially perpendicular directions;
   and/or
   wherein the first and/or second generally planar molecular layers (76, 78) are one atom thick;
   and/or
   wherein at least one of the first and second generally planar molecular layers (76, 78) is a graphene layer or a silicene layer.

4. A sensor arrangement according to any preceding claim, wherein the first and second generally planar molecular layers (76, 78) have substantially the same composition and/or structure; and, optionally, wherein the first and second generally planar molecular layers (76, 78) are both graphene layers or both silicene layers.

5. A sensor arrangement according to any of claims 1 to 3, wherein the first and second generally planar molecular layers (76, 78) have different compositions and/or structures.

6. A sensor arrangement according to any preceding claim, wherein the chemical species is a polymer with inhomogeneous charge distribution along its length; and, optionally, wherein the chemical species is DNA.

7. A sensor arrangement according to any preceding claim, wherein the first electrode (72) is covalently bonded to the second electrode (74) in the vicinity of the aperture (84) by $sp^2$ covalent bonding (82).

8. A DNA sequencing device comprising a sensor arrangement according to any preceding claim.

9. A method of measuring a property of a chemical species using an electrical sensor assembly (70), the electrical sensor assembly comprising:

   first and second electrodes (72, 74) comprising first and second generally planar molecular layers (76, 78), the first and second generally planar molecular layers each consisting of an array of covalently bonded atoms (80); and
   an aperture (84) through both the first and second molecular layers; and;
   the method comprising:

       passing the chemical species through the aperture;

applying a voltage across the first and second electrodes;
**characterised in that**
the first molecular layer is covalently bonded (82) to the second molecular layer to define said aperture; and
the method comprises measuring current flow between the first and second electrodes.

10. A method according to claim 9, wherein the first electrode (72) comprises the first molecular layer (76) and the second electrode (74) comprises the second molecular layer (78), wherein the first electrode generally extends in a first direction away from the aperture (84) and away from the second electrode (74), and wherein the current flow between the first and second electrodes flows at least partially via the covalent bonding (82) between the first and second molecular layers.

11. A method according to claim 9 or 10, wherein
the array of covalently bonded atoms (80) of one or both of the first and second generally planar molecular layers (76, 78) is a repeating structure, the repeating structure repeating in two substantially perpendicular directions;
and/or
wherein the first and/or second generally planar molecular layers (76, 78) are one atom thick;
and/or
wherein at least one of the first and second generally planar molecular layers (76, 78) is a graphene layer or a silicene layer.

12. A method according to any of claims 9 to 11, wherein the first and second generally planar molecular layers (76, 78) have substantially the same composition and/or structure; and, optionally, wherein the first and second generally planar molecular layers (76, 78) are both graphene layers or both silicene layers.

13. A method according to any of claims 9 to 11, wherein the first and second generally planar molecular layers (76, 78) have different compositions and/or structures.

14. A method according to any of claims 9 to 13,
wherein the chemical species is a polymer with inhomogeneous charge distribution along its length; and, optionally, wherein the chemical species is DNA;
and/or
wherein the first electrode (72) is covalently bonded to the second electrode (74) in the vicinity of the aperture (84) by $sp^2$ covalent bonding (82).

15. A method of DNA sequencing comprising a method according to any of claims 9 to 14.

**Patentansprüche**

1. Sensoranordnung, umfassend eine elektrische Sensorbaugruppe (70), geeignet zum Messen einer Eigenschaft einer chemischen Spezies, die elektrische Sensorbaugruppe umfassend:

eine erste und zweite Elektrode (72, 74), umfassend eine erste und zweite im Allgemeinen planare Molekularschicht (76, 78), wobei die erste und zweite im Allgemeinen planare Molekularschicht jeweils aus einer Anordnung von kovalent gebundenen Atomen (80) bestehen; und
eine Öffnung (84) durch sowohl die erste als auch die zweite Molekularschicht; und
wobei die Öffnung konfiguriert ist, zu ermöglichen, dass die chemische Spezies durch die Öffnung passiert; und
die Sensoranordnung ferner umfassend eine elektrische Leistungsversorgung (86), verbunden mit der ersten Elektrode und der zweiten Elektrode, wobei die Leistungsversorgung konfiguriert ist, eine Spannung über die erste Elektrode und die zweite Elektrode anzulegen;
**dadurch gekennzeichnet, dass**
die erste Molekularschicht an die zweite Molekularschicht kovalent gebunden (82) ist, um die Öffnung zu definieren; und
die Sensoranordnung ferner umfassend eine Strommessvorrichtung (88), konfiguriert zum Messen eines Stromflusses zwischen der ersten und zweiten Elektrode.

2. Sensoranordnung nach Anspruch 1, wobei die erste Elektrode (72) die erste Molekularschicht (76) umfasst und die zweite Elektrode (74) die zweite Molekularschicht (78) umfasst, wobei die erste Elektrode sich allgemein in einer ersten Richtung weg von der Öffnung (84) und weg von der zweiten Elektrode (74) erstreckt und wobei der Stromfluss zwischen der ersten und zweiten Elektrode mindestens teilweise über die kovalente Bindung (82) zwischen der ersten und zweiten Molekularschicht fließt.

3.  Sensoranordnung nach einem der vorstehenden Ansprüche, wobei
    die Anordnung von kovalent gebundenen Atomen (80) einer oder beider der ersten und der zweiten im Allgemeinen planaren Molekularschicht (76, 78) eine sich wiederholende Struktur ist, wobei die sich wiederholende Struktur sich in zwei im Wesentlichen senkrechten Richtungen wiederholt;
    und/oder
    wobei die erste und/oder die zweite im Allgemeinen planare Molekularschicht (76, 78) ein Atom dick sind;
    und/oder
    wobei mindestens eine der ersten und zweiten im Allgemeinen planaren Molekularschichten (76, 78) eine Graphenschicht oder eine Silicenschicht ist.

4.  Sensoranordnung nach einem der vorstehenden Ansprüche, wobei die erste und die zweite im Allgemeinen planare Molekularschicht (76, 78) im Wesentlichen die gleiche Zusammensetzung und/oder Struktur aufweisen; und wobei wahlweise die erste und zweite im Allgemeinen planare Molekularschicht (76, 78) beide Graphenschichten oder beide Silicenschichten sind.

5.  Sensoranordnung nach einem der Ansprüche 1 bis 3, wobei die erste und zweite im Allgemeinen planare Molekularschicht (76, 78) verschiedene Zusammensetzungen und/oder Strukturen aufweisen.

6.  Sensoranordnung nach einem der vorstehenden Ansprüche, wobei die chemische Spezies ein Polymer mit inhomogener Ladungsverteilung entlang seiner Länge ist; und wobei wahlweise die chemische Spezies DNA ist.

7.  Sensoranordnung nach einem der vorstehenden Ansprüche, wobei die erste Elektrode (72) in der Nähe der Öffnung (84) durch sp$^2$-Kovalentbindung (82) an die zweite Elektrode (74) kovalent gebunden ist.

8.  DNA-Sequenzierungsvorrichtung, umfassend eine Sensoranordnung nach einem der vorstehenden Ansprüche.

9.  Verfahren zum Messen einer Eigenschaft einer chemischen Spezies unter Verwendung einer elektrischen Sensorbaugruppe (70), die elektrische Sensorbaugruppe umfassend:

    eine erste und zweite Elektrode (72, 74), umfassend eine erste und zweite im Allgemeinen planare Molekularschicht (76, 78), wobei die erste und zweite im Allgemeinen planare Molekularschicht jeweils aus einer Anordnung von kovalent gebundenen Atomen (80) bestehen; und
    eine Öffnung (84) durch sowohl die erste als auch die zweite Molekularschicht; und
    das Verfahren umfassend:

    Passieren der chemischen Spezies durch die Öffnung;
    Anlegen einer Spannung über die erste und die zweite Elektrode;
    **dadurch gekennzeichnet, dass**
    die erste Molekularschicht an die zweite Molekularschicht kovalent gebunden (82) ist, um die Öffnung zu definieren; und
    das Verfahren umfasst, einen Stromfluss zwischen der ersten und zweiten Elektrode zu messen.

10. Verfahren nach Anspruch 9, wobei die erste Elektrode (72) die erste Molekularschicht (76) umfasst und die zweite Elektrode (74) die zweite Molekularschicht (78) umfasst, wobei die erste Elektrode sich allgemein in einer ersten Richtung weg von der Öffnung (84) und weg von der zweiten Elektrode (74) erstreckt und wobei der Stromfluss zwischen der ersten und zweiten Elektrode mindestens teilweise über die kovalente Bindung (82) zwischen der ersten und der zweiten Molekularschicht fließt.

11. Verfahren nach Anspruch 9 oder 10, wobei
    die Anordnung von kovalent gebundenen Atomen (80) einer oder beider der ersten und zweiten im Allgemeinen planaren Molekularschichten (76, 78) eine sich wiederholende Struktur ist, wobei die sich wiederholende Struktur sich in zwei im Wesentlichen senkrechten Richtungen wiederholt;
    und/oder
    wobei die erste und/oder zweite im Allgemeinen planare Molekularschicht (76, 78) ein Atom dick sind;

und/oder

wobei mindestens eine der ersten und zweiten im Allgemeinen planaren Molekularschicht (76, 78) eine Graphenschicht oder eine Silicenschicht ist.

**12.** Verfahren nach einem der Ansprüche 9 bis 11, wobei die erste und die zweite im Allgemeinen planare Molekularschicht (76, 78) im Wesentlichen die gleiche Zusammensetzung und/oder Struktur aufweisen; und wobei wahlweise die erste und zweite im Allgemeinen planare Molekularschicht (76, 78) beide Graphenschichten oder beide Silicenschichten sind.

**13.** Verfahren nach einem der Ansprüche 9 bis 11, wobei die erste und zweite im Allgemeinen planare Molekularschicht (76, 78) verschiedene Zusammensetzungen und/oder Strukturen aufweisen.

**14.** Verfahren nach einem der Ansprüche 9 bis 13,
wobei die chemische Spezies ein Polymer mit inhomogener Ladungsverteilung entlang seiner Länge ist; und wobei wahlweise die chemische Spezies DNA ist;
und/oder
wobei die erste Elektrode (72) in der Nähe der Öffnung (84) durch $sp^2$-Kovalentbindung (82) an die zweite Elektrode (74) kovalent gebunden ist.

**15.** Verfahren der DNA-Sequenzierung, umfassend ein Verfahren nach einem der Ansprüche 9 bis 14.

**Revendications**

**1.** Agencement de capteur comprenant un ensemble capteur électrique (70) approprié pour mesurer une propriété d'une espèce chimique, l'ensemble capteur électrique comprenant :

des première et seconde électrodes (72, 74) comprenant des première et seconde couches moléculaires généralement planes (76, 78), les première et seconde couches moléculaires généralement planes étant chacune constituée d'un réseau d'atomes liés de manière covalente (80) ; et
une ouverture (84) à travers à la fois les première et seconde couches moléculaires ; et
dans lequel l'ouverture est configurée pour permettre à l'espèce chimique de passer à travers l'ouverture ; et
l'agencement de capteur comprenant en outre une alimentation électrique (86) connectée à la première électrode et à la seconde électrode, dans lequel l'alimentation est configurée pour appliquer une tension aux bornes de la première électrode et de la seconde électrode ;
**caractérisé en ce que**
la première couche moléculaire est liée de manière covalente (82) à la seconde couche moléculaire pour définir ladite ouverture ; et
l'agencement de capteur comprenant en outre un dispositif de mesure du courant (88) configuré pour mesurer la circulation du courant entre les première et seconde électrodes.

**2.** Agencement de capteur selon 1, dans lequel la première électrode (72) comprend la première couche moléculaire (76) et la seconde électrode (74) comprend la seconde couche moléculaire (78), dans lequel la première électrode s'étend généralement dans une première direction s'éloignant de l'ouverture (84) et s'éloignant de la seconde électrode (74), et dans lequel la circulation du courant entre les première et seconde électrodes circule au moins partiellement par l'intermédiaire de la liaison covalente (82) entre les première et seconde couches moléculaires.

**3.** Agencement de capteur selon une quelconque revendication précédente, dans lequel
le réseau d'atomes liés de manière covalente (80) de l'une ou des deux des première et seconde couches moléculaires généralement planes (76, 78) est une structure répétitive, la structure répétitive se répétant dans deux directions substantiellement perpendiculaires ;
et/ou
dans lequel les première et/ou seconde couches moléculaires généralement planes (76, 78) ont une épaisseur d'un atome ;
et/ou
dans lequel au moins l'une des première et seconde couches moléculaires généralement planes (76, 78) est une couche de graphène ou une couche de silicène.

**4.** Agencement de capteur selon une quelconque revendication précédente, dans lequel les première et seconde couches moléculaires généralement planes (76, 78) ont substantiellement la même composition et/ou structure ; et, facultativement, dans lequel les première et seconde couches moléculaires généralement planes (76, 78) sont toutes deux des couches de graphène ou toutes deux des couches de silicène.

**5.** Agencement de capteur selon l'une quelconque des revendications 1 à 3, dans lequel les première et seconde couches moléculaires généralement planes (76, 78) ont des compositions et/ou structures différentes.

**6.** Agencement de capteur selon une quelconque revendication précédente, dans lequel l'espèce chimique est un polymère ayant une répartition non homogène des charges le long de sa longueur ; et, facultativement, dans lequel l'espèce chimique est de l'ADN.

**7.** Agencement de capteur selon une quelconque revendication précédente, dans lequel la première électrode (72) est liée de manière covalente à la seconde électrode (74) à proximité de l'ouverture (84) par une liaison covalente $sp^2$ (82).

**8.** Dispositif de séquençage d'ADN comprenant un agencement de capteur selon une quelconque revendication précédente.

**9.** Procédé de mesure d'une propriété d'une espèce chimique en utilisant un ensemble capteur électrique (70), l'ensemble capteur électrique comprenant :

des première et seconde électrodes (72, 74) comprenant des première et seconde couches moléculaires généralement planes (76, 78), les première et seconde couches moléculaires généralement planes étant chacune constituée d'un réseau d'atomes liés de manière covalente (80) ; et
une ouverture (84) à travers à la fois les première et seconde couches moléculaires ; et ;
le procédé comprenant :

le passage de l'espèce chimique à travers l'ouverture ;
l'application d'une tension aux bornes des première et seconde électrodes ;
**caractérisé en ce que**
la première couche moléculaire est liée de manière covalente (82) à la seconde couche moléculaire pour définir ladite ouverture ; et

le procédé comprend la mesure de la circulation du courant entre les première et seconde électrodes.

**10.** Procédé selon la revendication 9, dans lequel la première électrode (72) comprend la première couche moléculaire (76) et la seconde électrode (74) comprend la seconde couche moléculaire (78), dans lequel la première électrode s'étend généralement dans une première direction s'éloignant de l'ouverture (84) et s'éloignant de la seconde électrode (74), et dans lequel la circulation du courant entre les première et seconde électrodes circule au moins partiellement par l'intermédiaire de la liaison covalente (82) entre les première et seconde couches moléculaires.

**11.** Procédé selon la revendication 9 ou 10, dans lequel
le réseau d'atomes liés de manière covalente (80) de l'une ou des deux des première et seconde couches moléculaires généralement planes (76, 78) est une structure répétitive, la structure répétitive se répétant dans deux directions substantiellement perpendiculaires ;
et/ou
dans lequel les première et/ou seconde couches moléculaires généralement planes (76, 78) ont une épaisseur d'un atome ;
et/ou
dans lequel au moins l'une des première et seconde couches moléculaires généralement planes (76, 78) est une couche de graphène ou une couche de silicène.

**12.** Procédé selon l'une quelconque des revendications 9 à 11, dans lequel les première et seconde couches moléculaires généralement planes (76, 78) ont substantiellement la même composition et/ou structure ; et, facultativement, dans lequel les première et seconde couches moléculaires généralement planes (76, 78) sont toutes deux des couches de graphène ou toutes deux des couches de silicène.

**13.** Procédé selon l'une quelconque des revendications 9 à 11, dans lequel les première et seconde couches moléculaires généralement planes (76, 78) ont des compositions et/ou structures différentes.

**14.** Procédé selon l'une quelconque des revendications 9 à 13,
dans lequel l'espèce chimique est un polymère ayant une répartition non homogène des charges le long de sa longueur ; et, facultativement, dans lequel l'espèce chimique est de l'ADN ;
et/ou
dans lequel la première électrode (72) est liée de manière covalente à la seconde électrode (74) à proximité de l'ouverture (84) par une liaison covalente $sp^2$ (82).

**15.** Procédé de séquençage d'ADN comprenant un procédé selon l'une quelconque des revendications 9 à 14.

Figure 1

Prior Art

Figure 2

Figure 3

Figure 4

Figure 5

Figure 6

Figure 7

Figure 8

102

Figure 8a

102

104

Figure 8b

Figure 9A

Figure 9B

Figure 10

Figure 11

Figure 12

Figure 13

Figure 14

Figure 15

Figure 16

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 2010327847 A

## Non-patent literature cited in the description

- **S. LIU ; Q. ZHAO ; J. XU ; K. YAN ; H. PENG ; F. YANG et al.** Fast and controllable fabrication of suspended graphene nanopore devices. *Nanotechnology,* 02 March 2012, vol. 23, 085301-085301
- **A. T. KUAN ; J. A. GOLOVCHENKO.** Nanometer-thin solid-state nanopores by cold ion beam sculpting. *Applied Physics Letters,* 21 May 2012, vol. 100
- **C. J. RUSSO ; J. A. GOLOVCHENKO.** Atom-by-atom nucleation and growth of graphene nanopores. *Proceedings of the National Academy of Sciences of the United States of America,* 17 April 2012, vol. 109, 5953-5957
- **A. R. ROCHA ; V. M. GARCIA-SUAREZ ; S. W. BAILEY ; C. J. LAMBERT ; J. FERRER ; S. SANVITO.** Towards molecular spintronics. *Nat Mater,* 2005, vol. 4, 335-339